# EUROPEAN PATENT SPECIFICATION

(11) **EP 2 627 342 B1**
(45) Date of publication and mention of the grant of the patent: **24.08.2016**
(21) Application number: 11833476.2
(22) Date of filing: 14.10.2011
(51) Int. Cl.: A61K 35/34, A61K 35/54, C12N 5/077, C12N 5/074, A61P 9/00, A61P 9/10

(54) **CARDIAC INDUCED PLURIPOTENT STEM CELLS AND METHODS OF USE IN REPAIR AND REGENERATION OF MYOCARDIUM**
HERZANFALLINDUZIERTE PLURIPOTENTE STAMMZELLEN UND VERFAHREN ZU IHRER VERWENDUNG BEI DER REPARATUR UND REGENERATION DES HERZMUSKELS
CELLULES SOUCHES PLURIPOTENTES CARDIO-INDUITES ET PROCÉDÉS D'UTILISATION POUR LA RÉPARATION ET LA RÉGÉNÉRATION DU MYOCARDE

(30) Priority: 14.10.2010 US 393003 P
(43) Date of publication of application: 21.08.2013
(73) Proprietor: University Of Central Florida Research Foundation, Inc., Orlando FL 32826 (US)
(72) Inventor: SINGLA, Dinender, Oviedo FL 32765-8953 (US)
(74) Representative: Gardner, Rebecca Katherine
(86) International application number: PCT/US2011/056329
(87) International publication number: WO 2012/051515

(56) References cited:
- WO-A1-2006/002152
- WO-A1-2010/036923
- WO-A2-2010/059806
- US-A1- 2009 186 414
- US-A1- 2009 246 179
- US-A1- 2010 068 190
- SINGLA DINENDER K ET AL: "Induced Pluripotent Stem (iPS) Cells Repair and Regenerate Infarcted Myocardium", MOLECULAR PHARMACEUTICS, vol. 8, no. 5, September 2011 (2011-09), pages 1573-1581, XP002723017, ISSN: 1543-8384
- YAN B ET AL: "Transplanted induced pluripotent stem cells improve cardiac function and induce neovascularization in the infarcted hearts of db/db mice", MOLECULAR PHARMACEUTICS 20111003 AMERICAN CHEMICAL SOCIETY USA, vol. 8, no. 5, 3 October 2011 (2011-10-03) , pages 1602-1610, XP002723018, ISSN: 1543-8384
- T. J. NELSON ET AL: "Repair of Acute Myocardial Infarction by Human Stemness Factors Induced Pluripotent Stem Cells", CIRCULATION, vol. 120, no. 5, 4 August 2009 (2009-08-04), pages 408-416, XP055029219, ISSN: 0009-7322, DOI: 10.1161/CIRCULATIONAHA.109.865154
- MESSINA ELISA ET AL: "Isolation and expansion of adult cardiac stem cells from human and murine heart", CIRCULATION RESEARCH, AMERICAN HEART ASSOCIATION, INC, US, vol. 95, no. 9, 29 October 2004 (2004-10-29), pages 911-921, XP002477755, ISSN: 1524-4571, DOI: 10.1161/01.RES.0000147315.71699.51
- CESARE PESCHILE ET AL: "Stem Cells for Cardiomyocyte Regeneration: State of the Art", ANNALS OF THE NEW YORK ACADEMY OF SCIENCES, NEW YORK ACADEMY OF SCIENCES, US, vol. 1047, 1 June 2005 (2005-06-01), pages 376-385, XP007911937, ISSN: 0077-8923, DOI: 10.1196/ANNALS.1341.033 [retrieved on 2006-01-09]
- JOHN A. SCHOENHARD ET AL: "Stem Cell Therapy: Pieces of the Puzzle", JOURNAL OF CARDIOVASCULAR TRANSLATIONAL RESEARCH, vol. 3, no. 1, 19 November 2009 (2009-11-19), pages 49-60, XP55100139, ISSN: 1937-5387, DOI: 10.1007/s12265-009-9148-z
- Dinender K Singla: "Forum Review Article Embryonic Stem Cells in Cardiac Repair and Regeneration", Antioxid. Redox Signal, 1 August 2009 (2009-08-01), pages 1857-1863, XP55112853, Retrieved from the Internet: URL:http://online.liebertpub.com/doi/pdfpl us/10.1089/ars.2009.2491 [retrieved on 2014-04-08]

## Description

### I. RELATED APPLICATIONS

1. This application claims the benefit of U.S. Provisional Application No. 61/393,003 filed on October 14, 2010.

### II. STATEMENT OF GOVERNMENT SUPPORT

2. This invention was made with government support under Grant No. 1R01HL090646-01 awarded by the National Institutes of Health. The Government has certain rights in the invention.

### III. SUMMARY

3. Myocardial infarction (MI) leads to heart failure implicated by complex mechanisms of cardiac myocyte cell death, hypertrophy and fibrosis (Anversa, et al., Myocyte cell death and ventricular remodeling. Curr Opin Nephrol Hypertens 1997;6:169-176). Unfortunately, the common therapeutic preferences to inhibit further deterioration of MI leading to end stage heart failure are very restricted. Moreover, heart transplantation required to manage end stage heart failure patients is not readily available (Foo, et al., Death begets failure in the heart. J Clin Invest 2005;115:565-571). Therefore, new cellular therapies to repair and regenerate injured myocardium as well as to prevent the progression of adverse cardiac remodeling are under diligent examination. Thus far, cell transplantation in the mouse, rat, and human infarcted hearts has been studied employing a wide variety of cell types such as skeletal myoblasts, embryonic stem (ES) cells, c-kit positive cardiac stem cells, CD45^{+ve} bone marrow stem cells, mesenchymal stem cells (MSC), and hematopoietic stem cells (Haider & Ashraf, Bone marrow cell transplantation in clinical perspective. J Mol Cell Cardiol 2005;38:225-235; Kajstura, et al., Bone marrow cells differentiate in cardiac cell lineages after infarction independently of cell fusion. Circ Res 2005;96:127-137; Orlic, et al., Bone marrow cells regenerate infarcted myocardium. Nature 200;410:701-705; Singla, et al., Transplanted embryonic stem cells following mouse myocardial infarction inhibit apoptosis and cardiac remodeling. Am J Physiol Heart Circ Physiol 2007;293:H1308-H1314). Indeed, adult stem cells in clinical trials have furnished significant insights into cell transplantation; however, their effectiveness is still in question (Singla, Stem cells in the infarcted heart. J Cardiovasc Transl Res 2010;3:73-78). In contrast, ES cells have never reached in clinical trials due to ethical and additional concerns. Thus, the optimal cell types for cardiac regeneration, repair and remodeling for transplantation in the injured myocardium has yet to be identified. Recently identified, induced pluripotent stem (iPS) cells could be a potential cell type of choice to treat heart diseases but their basic mechanisms of cardiac repair and regeneration are largely unknown.

4. iPS cells generated from fibroblasts transfected with four factors (Oct3/4, Sox2, Klf4, and c-Myc; 4F-IPS cells) have the potential to differentiate into cardiac myocytes in the cell culture (Zhang, et al., Functional cardiomyocytes derived from human induced pluripotent stem cells. Circ Res 2009;104:e30-e41; Zhao & Daley, From fibroblasts to iPS cells: induced pluripotency by defined factors. J Cell Biochem 2008;105:949-955). In a recent pioneering study, Terzic and colleagues generated iPS cells with 3F (without c-MYC oncogene) and also established their potential to generate cardiac myocytes (Martinez-Fernandez, et al., iPS programmed without c-MYC yield proficient cardiogenesis for functional heart chimerism. Circ Res 2009;105:648-656). There is evidence that following transplantation in the infarcted heart, iPS cells emanated from fibroblast cells can develop into cardiac myocytes (Nelson, et al., Repair of acute myocardial infarction by human stemness factors induced pluripotent stem cells. Circulation 2009;120:408-416); however, there is no data available that determines the capacity to regenerate infarcted heart using iPS cells emanated from cardiac origin cell type sources such as H9c2 cells. The purpose of the present work was to generate rat cardiac iPS cells and to test their ability to differentiate into cardiac myocytes in the cell culture system as well as to repair (inhibit apoptosis and fibrosis) and regenerate the infarcted mouse heart.

### IV. BRIEF DESCRIPTION OF THE DRAWINGS

5. The accompanying drawings, which are incorporated in and constitute a part of this specification, illustrate several embodiments and together with the description illustrate the disclosed compositions and methods.
6. Figure 1(a) and (b) are photomicrographs of untransduced H9c2 cells whereas right panel shows transduced H9c2 cells forming ES cell like colony.
7. Figure 2 is a Western blot analysis, showing no expression of 4F (c-Myc, Oct3/4, Klf4 and Sox2) in H9c2 cells (left panel), and increased expression of 4Fs was present in transduced H9c2 cells (right panel).
8. Figures 3(a)-(c) are photomicrographs of alkaline phosphatase showing no positive staining in H9c2 cells (panel a), and stained ES cells (pinkish red, panel b, positive control) and stained iPS cells (panel c). 10x.
9. Figures 4(a)-(e) are confocal images demonstrate RFP-expressing ES cells and iPS cells (panel a and e), oct3/4 in ES cells and iPS cells (panel b and f), DAPI (panel c and g), and merge (panel d, and-h). 20x.
10. Figures 5(a)-(l) are photomicrographs of iPS cells form embryoid bodies (EBs) and differentiate into cardiomyocytes. EBs were differentiated for 17 days. Some of the small EBs showed large positive areas with anti-sarcomeric *a*-actin/Alexa 488 (panel f, ES cells-EBs, and panel j, iPS cells-EBs), and DAPI (c, g, k). H9c2 cells did not show *a*-actin staining (panel b). The merged images (panel d, h, and 1) shows co-expression of *a*-actin and RFP. 20x.
11. Figure 6(a)-(d) are photomicrographs of trypsinized iPS cells stained for cardiac myocytes with anti-sarcomeric a-actin/Alexa 488, (c) DAPI, and (d) merge. 160x.
12. Figures 7(a)-(l) are photomicrographs of iPS cells formed embryoid bodies (EBs) were trypsinized and stained with cardiac myocyte specific MHC, MF-20 (panel f, ES cells-EBs, and panel j, iPS cells-EBs), and DAPI (c, g, k). H9c2 cells did not show MF-20 staining (panel b). The merged images (panel d, h, and i) shows co-expression of MF-20, RFP and DAPI. 160x.
13. Figure 8 is a graph showing quantitative analysis of percentage positive cardiac myocytes in trypsinized EBs derived from ES and iPS cells seen in Figure 7. *p<0.001 vs H9c2 cells.
14. Figures 9(a)-(l) are photomicrographs of transplanted cardiac RFP-iPS or ES cells differentiated into cardiac myocytes post-MI. Cardiac myocyte specific anti-sarcomeric *a*-actin stained red shows cardiac myocytes (panel a, e, i and m), anti-RFP to identify donor cells (panel b, f, j and n), and DAPI (panel c, g, k and o). Merged images of all three stainings are shown in (panel d, h, l and p). 40x.
15. Figure 10 is a graph showing quantitative analysis of newly differentiated cardiac myocytes following transplantation at 2 weeks post-MI, seen in Figure 7. *p<0.001 vs MI and H9c2 cells.
16. Figures 11(a)-(l) are photomicrographs of transplanted cardiac RFP-iPS or ES cells differentiated into cardiac myocytes post-MI. Cells are stained with anti-sarcomeric *a*-actin (panel a, and f), anti-RFP (panel b, and g), connexin-43 (panel c and h) to identify formation of gap junction between donor derived cardiac myocytes and native myocardium (panel b, and g), and DAPI (panel d, and i). Merged images are shown in (panel e and j). a-e, 40x, and f-j, 120x.
17. Figures 12(a)-(l) are photomicrographs of transplanted iPS cells inhibit apoptosis at 2-weeks post-MI. Representative photomicrographs of TUNEL stained apoptotic nuclei are depicted (panel a, d, g and j), total nuclei stained with DAPI (panel b, e, h and k) and merged nuclei (panel c, f, i and 1). 40x.
18. Figure 13 is histogram showing quantitative percentage of apoptotic nuclei. *p<0.05 vs MI and H9c2 cells.
19. Figures 14(a)-(l) are photomicrographs of transplanted iPS cells apoptosis at 2-weeks post-MI, showing anti-sarcomeric *a*-actin (panel a, and f), TUNEL (panel b, and g), caspase-3 immunolabeling, (panel c, and h), and DAPI (panel d, and i). Merged images are shown in (panel e and j). a-e, 40x, and lower level f-j, 160x.

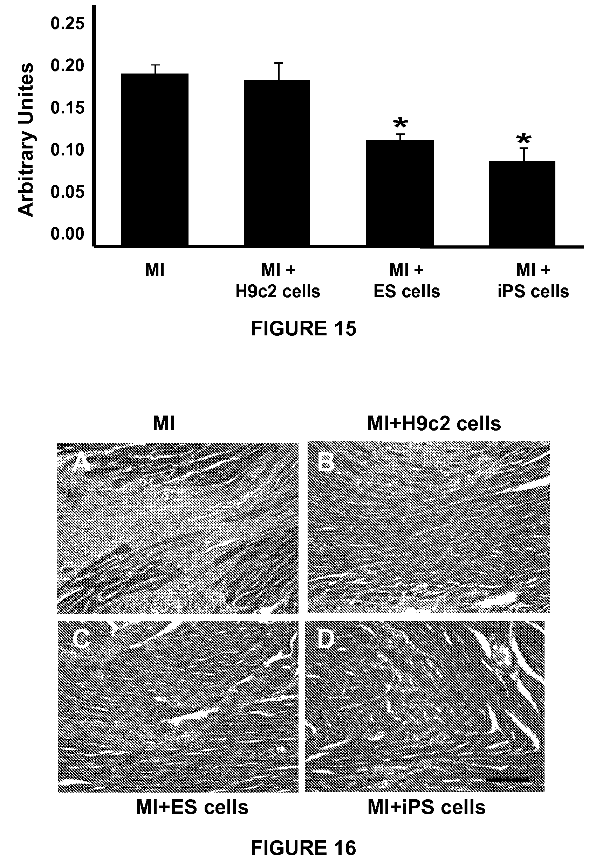

20. Figure 15 is histogram showing quantitative caspase-3 activity. *p<0.05 vs MI and H9c2 cells.
21. Figures 16(a)-(d) are representative photomicrographs from Masson's trichrome stained heart sections with and without iPS cells transplantation post-MI; MI+cell culture medium (panel a), MI+H9c2 cells (panel b), MI+ES cells (panel c), MI+iPS cells (panel d); (20x).
22. Figure 17 is histogram showing quantitatively less fibrosis in MI+iPS or ES cell groups compare with MI+cell culture medium and MI+H9c2 cells groups. (*p<0.05).
23. Figure 18 is a series of H&E stained heart section with and without transplanted stem cells at 2 weeks post-MI shows no evidence of teratoma formation. (1.25x).

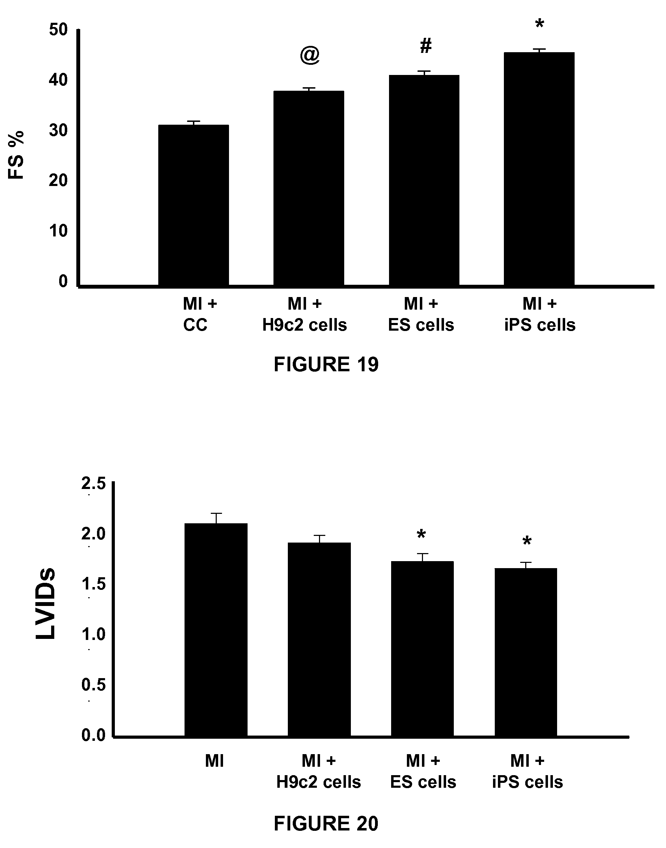

24. Figure 19 is graph showing transplanted iPS cells improves cardiac function. Average echocardiographic fractional shortening (FS) for treatment groups. *p<0.05 vs MI, H9c2 and ES cells, # p<0.05 vs MI and H9c2 cells, @p<0.05 vs MI.
25. Figure 20 is graph showing transplanted iPS cells improves cardiac function. Left ventricular interior diastolic diameter systolically (LVIDs) for different treatment groups. *p<0.05 vs MI, and &p=Non-significant.

### V. DETAILED DESCRIPTION

26. Before the present compounds, compositions, articles, devices, and/or methods are disclosed and described, it is to be understood that they are not limited to specific synthetic methods or specific recombinant biotechnology methods unless otherwise specified, or to particular reagents unless otherwise specified, as such may, of course, vary.

The present invention provides cardiac induced pluripotent stem cells for use in the treatment of damaged cardiac tissue due to cardiac dysfunction by repairing or regenerating said damaged cardiac tissue in a subject in need thereof,
wherein the cardiac induced pluripotent stem cells are derived from cardiofibroblasts, cardiomyoblasts, or H9c2 cardiomyoblasts that stably express inserted stem-cell like factors,
wherein the inserted stem-cell like factors comprise Oct-3/4, KIf4, Sox2, and c-Myc, and
wherein cDNA sequences for the stem-cell like factors are inserted using a plasmid vector.

The present invention also provides cardiac induced pluripotent stem cells derived from cardiofibroblasts, cardiomyoblasts, or H9c2 cardiomyoblasts that stably express inserted stem-cell like factors,
wherein the inserted stem-cell like factors comprise Oct-3/4, KIf4, Sox2, and c-Myc, and
wherein cDNAs for the stem-cell like factors are inserted via a plasmid vector; and
a method of making said cardiac induced pluripotent stem cells, comprising:
obtaining cardiofibroblasts, cardiomyoblasts, or H9c2 cardiomyoblasts,
inserting into the cardiofibroblasts, cardiomyoblasts, or H9c2 cardiomyoblasts a plasmid vector comprising cDNA sequences for stem-cell like factors, wherein the stem-cell like factors comprise Oct-3/4, KIf4, Sox2, and c-Myc, and
stably expressing the inserted stem-cell like factors, thereby converting the cardiofibroblasts, cardiomyoblasts, or H9c2 cardiomyoblasts into cardiac induced pluripotent stem cells.

### A. Definitions

27. As used in the specification and the appended claims, the singular forms "a," "an" and "the" include plural referents unless the context clearly dictates otherwise. Thus, for example, reference to "a pharmaceutical carrier" includes mixtures of two or more such carriers, and the like.

28. Ranges can be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that when a value is disclosed that "less than or equal to" the value, "greater than or equal to the value" and possible ranges between values are also disclosed, as appropriately understood by the skilled artisan. For example, if the value "10" is disclosed the "less than or equal to 10"as well as "greater than or equal to 10" is also disclosed. It is also understood that the throughout the application, data is provided in a number of different formats, and that this data, represents endpoints and starting points, and ranges for any combination of the data points. For example, if a particular data point "10" and a particular data point 15 are disclosed, it is understood that greater than, greater than or equal to, less than, less than or equal to, and equal to 10 and 15 are considered disclosed as well as between 10 and 15.

29. In this specification and in the claims which follow, reference will be made to a number of terms which shall be defined to have the following meanings:

30. "Optional" or "optionally" means that the subsequently described event or circumstance may or may not occur, and that the description includes instances where said event or circumstance occurs and instances where it does not.

31. Throughout this application, various publications are referenced.

### B. Methods of reducing, repairing and regenerating damaged cardiac tissue resulting from cardiac dysfunction

32. Herein disclosed are methods of reducing, repairing, and/or regenerating damaged cardiac tissue due to cardiac dysfunction in a subject in need thereof comprising administering to the subject cardiac induced pluripotent stems cells derived from a cardiac cell type. It is understood and herein contemplated that cardiac dysfunction can be caused by many events such as congenital cardiac defects as well as ischemia/reperfusion events. Therefore, in one aspect, for example, disclosed herein are methods of reducing, repairing, and/or regenerating damaged cardiac tissue due to cardiac dysfunction wherein the cardiac dysfunction is ischemia/reperfusion event.

33. As used herein, "ischemia" refers to a deficiency of blood in a part, usually due to functional constriction or actual obstruction of a blood vessel. Such a deficiency result in an infarct, an area of cell death in a tissue due to local ischemia resulting from obstruction of circulation to the area, most commonly by a thrombus or embolus. When the constriction or obstruction is removed, and blood flow restored reperfusion has occurred. Although blood flow is restored, the reperfusion can also result in adverse effects of the restoration of blood flow following an ischemic episode, including cellular swelling and necrosis, apoptosis, edema, hemorrhage, the no-reflow phenomenon, and tissue damage by free oxygen radicals. Thus, one manifestation of reducing ischemia/reperfusion injury is reducing infarct size. Accordingly, in one aspect, disclosed herein are methods of reducing infarct size following injury due to cardiac dysfunction in a subject comprising administering to the subject cardiac induced pluripotent stem cells derived from a cardiac cell type.

34. It is understood that there are many known causes of ischemia/reperfusion injury. For example, an ischemic/reperfusion injury can result from ischemia reperfusion event such as myocardial infraction, myocardial ischemia, myocardial reperfusion, subendocardial ischemia, Takayasu's arteritis, atrial fibrillation, hemorrhagic strokes (including strokes resulting from aneurysm and arteriovenous malformation), and transient ischemic attack), cardiac surgery where a heart lung machine is used such as Coronary artery bypassing, and preservation of organs for transplant. For example, and in one aspect, also disclosed herein are methods of reducing, repairing, or regenerating damaged cardiac tissue due to cardiac dysfunction comprising administering to a subject cardiac induced pluripotent stem cells; wherein the cardiac dysfunction is ischemia reperfusion injury; and wherein the ischemia/reperfusion injury occurs following an ischemia/reperfusion event selected from the group consisting of myocardial ischemia, myocardial reperfusion, subendocardial ischemia, Takayasu's arteritis, stroke, ischemia strokes, atrial fibrillation, hemorrhagic strokes, transient ischemia attack, ischemic/reperfusion event occurring during cardiac surgery where a heart lung machine is used such as Coronary artery bypassing, and ischemic/reperfusion events occurring during the preservation of organs for transplant.

35. Cardiac dysfunction can also be the result of congenital defects in a subject that result in damaged cardiac tissue or tissue in need of corrective repair. For example, disclosed herein are methods of reducing, repairing, and/or regenerating damaged cardiac tissue due to cardiac dysfunction comprising administering to a subject in need thereof cardiac induced pluripotent stem cells derived from cardiac tissue, wherein the cardiac dysfunction was a congenital cardiac defect such as, for example, hypoplasia and/or pentalogy of Cantrell.

36. By "treatment" and "treating" is meant the medical management of a subject with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder. It is understood that treatment, while intended to cure, ameliorate, stabilize, or prevent a disease, pathological condition, or disorder, need not actually result in the cure, ameliorization, stabilization or prevention. It is understood and herein contemplated that "treatment" does not necessarily refer to a cure of the disease or condition nor a complete prevention of infarct, but can involve, for example, an improvement in the outlook of an ischemia/reperfusion injury. The effects of treatment can be measured or assessed as described herein and as known in the art as is suitable for the disease, pathological condition, or disorder involved. Such measurements and assessments can be made in qualitative and/or quantitative terms. Thus, for example, characteristics or features of a disease, pathological condition, or disorder and/or symptoms of a disease, pathological condition, or disorder can be reduced to any effect or to any amount.

37. "Reducing," "reduce," or "reduction" in the context of a disease or condition herein refers to a decrease in the cause, symptoms, or effects of a disease or condition. Therefore, in the disclosed methods, "reducing" can refer to a 10%, 20%, 30%, 40%, 50%, 60%, 70%, 80%, 90%, or 100% decrease in the amount of injury due to ischemia/reperfusion including but not limited to infarct size.

38. Unfortunately, cardiac dysfunction can occur in subjects who are unaware of the impending dysfunction such as, for example, an infarction. Often the first indication to such individuals is the dysfunction itself, such as a ischemic/reperfusion event. In such individuals, there is a need to reduce the potential cardiac tissue injury. Thus, it is herein contemplated that disclosed methods can be used to reduce, repair, or regenerate cardiac tissue damage following the cardiac dysfunction. For example, disclosed are methods of reducing, repairing, and/or regenerating cardiac damage due to a cardiac dysfunction such as, for example an ischemia/reperfusion event, in a subject in need thereof comprising administering to the subject cardiac induced pluripotent stem cells (iPS) derived from a cardiac cell type, wherein the iPS are administered within 24 hours following the cardiac dysfunction injury. It is understood that the more quickly the iPS can be administered following the cardiac dysfunction event, the less the likelihood of injury and subsequently the greater the potential reduction in damage such as, for example, a decrease in infarct size. Thus, disclosed herein are methods wherein the cardiac induced pluripotent stem cells are administered within 24, 12, 6, 2, 1 hour(s), 30, 15, 10, 5 minutes following the cardiac dysfunction event or injury.

39. It is understood that administration of the cardiac induced pluripotent stem cells (iPS) can occur at any time between 5 minutes and 24 hours following the cardiac dysfunction event or injury. Where the dysfunction is a ischemia/reperfusion event, it is also understood that ischemia and reperfusion are not only physiologically different events, but do not necessarily occur at the same time. As ischemia refers to deficiency of blood to a part typically due to a thrombus or embolus and reperfusion injury results when the obstruction or constriction is removed, it is possible and desirable to reduce cardiac dysfunction injury as the cardiac dysfunction is occurring, for example, during a ischemia/reperfusion event. Thus, for example, iPS can be administered during the ischemia or alternatively after the ischemia, but before reperfusion has occurred, or alternatively after the ischemia and at the time of reperfusion. Similarly iPS can be administered during the occurrence of any cardiac dysfunction event. Thus, disclosed herein are methods wherein iPS is administered during the cardiac dysfunction event.

40. It is understood and herein contemplated that reduction in cardiac damage due to a cardiac dysfunction will occur in individuals where the iPS are administered before the cardiac dysfunction. Thus, it is understood and contemplated herein that individuals at risk for or having a history of cardiac dysfunction can decrease the risk of further damage in future events by taking the iPS prophylactically. It is also understood that many cardiac dysfunctions, such as, ischemia/reperfusion events like myocardial infarction, have early warning symptoms preceding the actual event which when recognized can allow the subject to seek immediate treatment. Even if there is cardiac tissue injury caused by future cardiac dysfunction events, it is contemplated that the prophylactic administration of iPS can reduce the cardiac tissue injury such as, for example, reduce infarct size. For example, disclosed herein are methods of reducing cardiac tissue injury in a subject in need thereof comprising administering to the subject iPS, wherein the iPS are administered at least 30 minutes before the cardiac dysfunction event. Thus, disclosed herein are methods wherein iPS are administered 15, 30 minutes, 1, 2, 6, 12, 24 hour(s), 2, 3 days, 1, or 2 weeks or any time point in between before the cardiac dysfunction event.

### C. Compositions

41. Disclosed are the components to be used to prepare the disclosed compositions as well as the compositions themselves to be used within the methods disclosed herein. These and other materials are disclosed herein, and it is understood that when combinations, subsets, interactions, groups, etc. of these materials are disclosed that while specific reference of each various individual and collective combinations and permutation of these compounds may not be explicitly disclosed, each is specifically contemplated and described herein. For example, if a particular stemness factor (e.g., Oct3/4, KIf4, Sox2, and c-Myc) or cardiac induced pluripotent stem cell is disclosed and discussed and a number of modifications that can be made to a number of molecules including the stemness factor (e.g., Oct3/4, KIf4, Sox2, and c-Myc) or cardiac induced pluripotent stem cell are discussed, specifically contemplated is each and every combination and permutation of stemness factor (e.g., Oct3/4, KIf4, Sox2, and c-Myc) or cardiac induced pluripotent stem cell and the modifications that are possible unless specifically indicated to the contrary. Thus, if a class of molecules A, B, and C are disclosed as well as a class of molecules D, E, and F and an example of a combination molecule, A-D is disclosed, then even if each is not individually recited each is individually and collectively contemplated meaning combinations, A-E, A-F, B-D, B-E, B-F, C-D, C-E, and C-F are considered disclosed. Likewise, any subset or combination of these is also disclosed. Thus, for example, the subgroup of A-E, B-F, and C-E would be considered disclosed. This concept applies to all aspects of this application including, but not limited to, steps in methods of making and using the disclosed compositions. Thus, if there are a variety of additional steps that can be performed it is understood that each of these additional steps can be performed with any specific embodiment or combination of embodiments of the disclosed methods.

42. In one aspect, disclosed herein are cardiac induced pluripotent stems cells (iPS). As discussed and disclosed herein, cardiac induced pluripotent stem cells are induced stem cells that are derived from cardiac cell types that have stemness factors inserted and expressed within them. Stemness factors are known in the art and include but are not limited to Oct3/4, KIf4, Sox2, and c-Myc. Thus, for example, disclosed herein are iPS comprising a cardiac cell type having a stable vector inserted in the cell, and wherein the vector further comprises Oct3/4, KIf4, Sox2, and c-Myc. The stemness factors can be operably linked to each other. For example, disclosed herein are cardiac induced pluripotent stem cells comprising a vector which further comprises the stemness factors, Oct3/4, KIf4, Sox2, and c-Myc, wherein the stemness factors are operably linked through the self-cleaving 2A sequence of foot and mouth disease virus. The disclosed cardiac induced pluripotent stem cells can be used in any of the methods disclosed herein. For example, disclosed herein are methods of reducing, repairing, and/or regenerating damaged cardiac tissue due to cardiac dysfunction in a subject in need thereof comprising administering to the subject iPS derived from a cardiac cell type, wherein the iPS comprise a vector which further comprises the stemness factors, Oct3/4, KIf4, Sox2, and c-Myc.

43. It is contemplated herein that the vector contained within the cardiac cell types and for use in expressing stemness factors can be any vector appropriate for stably expressing genes in a cell. For example, the vector can be apBS-2A vector.

44. Cardiac cell types that can be used in to produce the iPS disclosed herein can be any cardiac cell type, including but not limited to cardiomyoblasts (e.g., cardiomyocytes) and cardiofibroblasts. An example of a cardiomyoblast that can be used for the iPS disclosed herein is the H9c2 cardiomyoblast cell. Moreover, the myocytes and fibroblasts can come from any source species of mammal including but not limited to human, non-human primate, simian, bovine, porcine, equine, rabbit, canine, feline, rat, guinea pig, and mouse.

45. Where cells are to be delivered to a subject, it is understood that the more immunologically similar the source of the cardiac cell type is, the less likely immunological rejection or graft versus host disease will occur. Accordingly, it is contemplated herein that the source of the cardiac cell types which are ultimately induced to form cardiac induced pluripotent stem cells can be an autologous, allogeneic, or syngeneic source. For example, disclosed herein are methods of reducing, repairing, and/or regenerating damaged cardiac tissue due to cardiac dysfunction in a subject in need thereof comprising administering to the subject iPS derived from a cardiac cell type, wherein the cardiac cell type is obtained from an autologous, allogeneic, or syngeneic source.

46. Also disclosed herein are methods of making the cardiac induced pluripotent stem (iPS) cells described. In one aspect, the disclosed methods of making iPS comprise inserting a vector which comprises stemness factors into a cardiac cell type. For example, disclosed herein are methods of generating cardiac induced pluripotent stem cells, comprising the steps of 1) inserting one or more stem-cell like factors into a cardiac cell type, wherein the stem-cell like factors comprise Oct3/4, KIf4, Sox2 and c-Myc or any combination thereof; and 2) obtaining the cardiac induced pluripotent stem cells stably expressing the stem-cell like factors in the cardiac cell type. In a further aspect, the disclosed methods of making iPS can comprise comprising independently inserting cDNAs of the stem-cell like factors Oct3/4 and KIf4 into separate vectors. In still a further aspect, the disclosed methods of making iPS can comprise a) isolating the KIf4-2A sequence; b)inserting the KIf4-2A sequence into the Oct-3/4 vector in the same reading frame forming a Oct-3/4- KIf4-2A vector; and c) fusing the Oct-3/4- KIf4-2A vector to Sox2 cDNA with a stop codon forming a Oct-3/4- KIf4-2A- Sox2 vector. In yet a further aspect disclosed herein are methods of making iPS further comprising removing the Oct-3/4- KIf4-2A- Sox2 and inserting it into a pCX-EGFP vector. The method of making iPS can still further comprise inserting c-Myc cDNA into the vector. The c-Myc can be inserted in any place of the vector appropriate to operatively link c-Myc to the other stemness factors and retain the ability to be operatively expressed. For example, c-Myc can replace EGFP in a pCX-EGFP vector.

### 1. Delivery of the compositions to cells

47. There are a number of compositions and methods which can be used to deliver nucleic acids to cells, either in vitro or in vivo. These methods and compositions can largely be broken down into two classes: viral based delivery systems and non-viral based delivery systems. For example, the nucleic acids can be delivered through a number of direct delivery systems such as, electroporation, lipofection, calcium phosphate precipitation, plasmids, viral vectors, viral nucleic acids, phage nucleic acids, phages, cosmids, or via transfer of genetic material in cells or carriers such as cationic liposomes. Appropriate means for transfection, including viral vectors, chemical transfectants, or physico-mechanical methods such as electroporation and direct diffusion of DNA, are described by, for example, Wolff, J. A., et al., Science, 247, 1465-1468, (1990); and Wolff, J. A. Nature, 352, 815-818, (1991). Such methods are well known in the art and readily adaptable for use with the compositions and methods described herein. In certain cases, the methods will be modified to specifically function with large DNA molecules. Further, these methods can be used to target certain diseases and cell populations by using the targeting characteristics of the carrier.

### a) Nucleic acid based delivery systems

48. Transfer vectors can be any nucleotide construction used to deliver genes into cells (e.g., a plasmid). In the uses and methods of the present invention the cDNA sequences for the stem-cell like factors are inserted using a plasmid vector. However, other strategies to deliver genes are known in the art, e.g., genes may be delivered as part of recombinant retrovirus or adenovirus (Ram et al. Cancer Res. 53:83-88, (1993)).

49. As used herein, plasmid or viral vectors are agents that transport the disclosed nucleic acids, such as the genes encoding the stemness factors disclosed herein (e.g., Oct-3/4, KIf4-2A, Sox2 and c-Myc) into the cell without degradation and include a promoter yielding expression of the gene in the cells into which it is delivered. The following passages describe examples of delivery systems which could be used as alternative methods of delivering genes to cells to the delivery method of the present invention.

Viral vectors are, for example, Adenovirus, Adeno-associated virus, Herpes virus, Vaccinia virus, Polio virus, foot and mouth disease virus, AIDS virus, neuronal trophic virus, Sindbis and other RNA viruses, including these viruses with the HIV backbone. Also preferred are any viral
families which share the properties of these viruses which make them suitable for use as vectors. Retroviruses include Murine Maloney Leukemia virus, MMLV, and retroviruses that express the desirable properties of MMLV as a vector. Retroviral vectors are able to carry a larger genetic payload, i.e., a transgene or marker gene, than other viral vectors, and for this reason are a commonly used vector. However, they are not as useful in non-proliferating cells. Adenovirus vectors are relatively stable and easy to work with, have high titers, and can be delivered in aerosol formulation, and can transfect non-dividing cells. Pox viral vectors are large and have several sites for inserting genes, they are thermostable and can be stored at room temperature. An example is a viral vector which has been engineered so as to suppress the immune response of the host organism, elicited by the viral antigens. Preferred vectors of this type will carry coding regions for Interleukin 8 or 10. It is contemplated herein that the vector contained within the cardiac cell types and for use in expressing stemness factors can be any vector appropriate for stably expressing genes in a cell. For example, the vector can be apBS-2A vector.

50. Viral vectors can have higher transaction (ability to introduce genes) abilities than chemical or physical methods to introduce genes into cells. Typically, viral vectors contain, nonstructural early genes, structural late genes, an RNA polymerase III transcript, inverted terminal repeats necessary for replication and encapsidation, and promoters to control the transcription and replication of the viral genome. When engineered as vectors, viruses typically have one or more of the early genes removed and a gene or gene/promotor cassette is inserted into the viral genome in place of the removed viral DNA. Constructs of this type can carry up to about 8 kb of foreign genetic material. The necessary functions of the removed early genes are typically supplied by cell lines which have been engineered to express the gene products of the early genes in trans.

### (1) Retroviral Vectors

51. A retrovirus is an animal virus belonging to the virus family of Retroviridae, including any types, subfamilies, genus, or tropisms. A retrovirus is essentially a package which has packed into it nucleic acid cargo. The nucleic acid cargo carries with it a packaging signal, which ensures that the replicated daughter molecules will be efficiently packaged within the package coat. In addition to the package signal, there are a number of molecules which are needed in cis, for the replication, and packaging of the replicated virus. Typically a retroviral genome, contains the gag, pol, and env genes which are involved in the making of the protein coat. It is the gag, pol, and env genes which are typically replaced by the foreign DNA that it is to be transferred to the target cell. Retrovirus vectors typically contain a packaging signal for incorporation into the package coat, a sequence which signals the start of the gag transcription unit, elements necessary for reverse transcription, including a primer binding site to bind the tRNA primer of reverse transcription, terminal repeat sequences that guide the switch of RNA strands during DNA synthesis, a purine rich sequence 5' to the 3' LTR that serve as the priming site for the synthesis of the second strand of DNA synthesis, and specific sequences near the ends of the LTRs that enable the insertion of the DNA state of the retrovirus to insert into the host genome. The removal of the gag, pol, and env genes allows for about 8 kb of foreign sequence to be inserted into the viral genome, become reverse transcribed, and upon replication be packaged into a new retroviral particle. This amount of nucleic acid is sufficient for the delivery of a one to many genes depending on the size of each transcript. It is preferable to include either positive or negative selectable markers along with other genes in the insert.

52. Since the replication machinery and packaging proteins in most retroviral vectors have been removed (gag, pol, and env), the vectors are typically generated by placing them into a packaging cell line. A packaging cell line is a cell line which has been transfected or transformed with a retrovirus that contains the replication and packaging machinery, but lacks any packaging signal. When the vector carrying the DNA of choice is transfected into these cell lines, the vector containing the gene of interest is replicated and packaged into new retroviral particles, by the machinery provided in cis by the helper cell. The genomes for the machinery are not packaged because they lack the necessary signals.

### (2) Adenoviral Vectors

53. The construction of replication-defective adenoviruses has been described (Berkner et al., J. Virology 61:1213-1220 (1987); Massie et al., Mol. Cell. Biol. 6:2872-2883 (1986); Haj-Ahmad et al., J. Virology 57:267-274 (1986); Davidson et al., J. Virology 61:1226-1239 (1987); Zhang "Generation and identification of recombinant adenovirus by liposome-mediated transfection and PCR analysis" BioTechniques 15:868-872 (1993)). The benefit of the use of these viruses as vectors is that they are limited in the extent to which they can spread to other cell types, since they can replicate within an initial infected cell, but are unable to form new infectious viral particles. Recombinant adenoviruses have been shown to achieve high efficiency gene transfer after direct, in vivo delivery to airway epithelium, hepatocytes, vascular endothelium, CNS parenchyma and a number of other tissue sites (Morsy, J. Clin. Invest. 92:1580-1586 (1993); Kirshenbaum, J. Clin. Invest. 92:381-387 (1993); Roessler, J. Clin. Invest. 92:1085-1092 (1993); Moullier, Nature Genetics 4:154-159 (1993); La Salle, Science 259:988-990 (1993); Gomez-Foix, J. Biol. Chem. 267:25129-25134 (1992); Rich, Human Gene Therapy 4:461-476 (1993); Zabner, Nature Genetics 6:75-83 (1994); Guzman, Circulation Research 73:1201-1207 (1993); Bout, Human Gene Therapy 5:3-10 (1994); Zabner, Cell 75:207-216 (1993); Caillaud, Eur. J. Neuroscience 5:1287-1291 (1993); and Ragot, J. Gen. Virology 74:501-507 (1993)). Recombinant adenoviruses achieve gene transduction by binding to specific cell surface receptors, after which the virus is internalized by receptor-mediated endocytosis, in the same manner as wild type or replication-defective adenovirus (Chardonnet and Dales, Virology 40:462-477 (1970); Brown and Burlingham, J. Virology 12:386-396 (1973); Svensson and Persson, J. Virology 55:442-449 (1985); Seth, et al., J. Virol. 51:650-655 (1984); Seth, et al., Mol. Cell. Biol. 4:1528-1533 (1984); Varga et al., J. Virology 65:6061-6070 (1991); Wickham et al., Cell 73:309-319 (1993)).

54. A viral vector can be one based on an adenovirus which has had the E1 gene removed and these virons are generated in a cell line such as the human 293 cell line. In another example both the E1 and E3 genes are removed from the adenovirus genome.

### (3) Adeno-associated viral vectors

55. Another type of viral vector is based on an adeno-associated virus (AAV). This defective parvovirus is a preferred vector because it can infect many cell types and is nonpathogenic to humans. AAV type vectors can transport about 4 to 5 kb and wild type AAV is known to stably insert into chromosome 19. Vectors which contain this site specific integration property are preferred. An example of this type of vector is the P4.1 C vector produced by Avigen, San Francisco, CA, which can contain the herpes simplex virus thymidine kinase gene, HSV-tk, and/or a marker gene, such as the gene encoding the green fluorescent protein, GFP.

56. In another type of AAV virus, the AAV contains a pair of inverted terminal repeats (ITRs) which flank at least one cassette containing a promoter which directs cell-specific expression operably linked to a heterologous gene. Heterologous in this context refers to any nucleotide sequence or gene which is not native to the AAV or B 19 parvovirus.

57. Typically the AAV and B 19 coding regions have been deleted, resulting in a safe, noncytotoxic vector. The AAV ITRs, or modifications thereof, confer infectivity and site-specific integration, but not cytotoxicity, and the promoter directs cell-specific expression.

58. The disclosed vectors thus provide DNA molecules which are capable of integration into a mammalian chromosome without substantial toxicity.

59. The inserted genes in viral and retroviral usually contain promoters, and/or enhancers to help control the expression of the desired gene product. A promoter is generally a sequence or sequences of DNA that function when in a relatively fixed location in regard to the transcription start site. A promoter contains core elements required for basic interaction of RNA polymerase and transcription factors, and may contain upstream elements and response elements.

### (4) Large payload viral vectors

60. Molecular genetic experiments with large human herpesviruses have provided a means whereby large heterologous DNA fragments can be cloned, propagated and established in cells permissive for infection with herpesviruses (Sun et al., Nature genetics 8: 33-41, 1994; Cotter and Robertson,.Curr Opin Mol Ther 5: 633-644, 1999). These large DNA viruses (herpes simplex virus (HSV) and Epstein-Barr virus (EBV), have the potential to deliver fragments of human heterologous DNA > 150 kb to specific cells. EBV recombinants can maintain large pieces of DNA in the infected B-cells as episomal DNA. Individual clones carried human genomic inserts up to 330 kb appeared genetically stable The maintenance of these episomes requires a specific EBV nuclear protein, EBNA1, constitutively expressed during infection with EBV. Additionally, these vectors can be used for transfection, where large amounts of protein can be generated transiently in vitro. Herpesvirus amplicon systems are also being used to package pieces of DNA > 220 kb and to infect cells that can stably maintain DNA as episomes.

61. Other useful systems include, for example, replicating and host-restricted non-replicating vaccinia virus vectors.

### b) Non-nucleic acid based systems

62. The disclosed compositions can be delivered to the target cells in a variety of ways. For example, the compositions can be delivered through electroporation, or through lipofection, or through calcium phosphate precipitation. The delivery mechanism chosen will depend in part on the type of cell targeted and whether the delivery is occurring for example in vivo or in vitro.

63. Thus, the compositions can comprise, in addition to the disclosed vectors for example, lipids such as liposomes, such as cationic liposomes (e.g., DOTMA, DOPE, DC-cholesterol) or anionic liposomes. Liposomes can further comprise proteins to facilitate targeting a particular cell, if desired. Administration of a composition comprising a compound and a cationic liposome can be administered to the blood afferent to a target organ or inhaled into the respiratory tract to target cells of the respiratory tract. Regarding liposomes, see, e.g., Brigham et al. Am. J. Resp. Cell. Mol. Biol. 1:95-100 (1989); Felgner et al. Proc. Natl. Acad. Sci USA 84:7413-7417 (1987); U.S. Pat. No.4,897,355. Furthermore, the compound can be administered as a component of a microcapsule that can be targeted to specific cell types, such as macrophages, or where the diffusion of the compound or delivery of the compound from the microcapsule is designed for a specific rate or dosage.

64. In the methods described above which include the administration and uptake of exogenous DNA into the cells of a subject (i.e., gene transduction or transfection), delivery of the compositions to cells can be via a variety of mechanisms. As one example, delivery can be via a liposome, using commercially available liposome preparations such as LIPOFECTIN, LIPOFECTAMINE (GIBCO-BRL, Inc., Gaithersburg, MD), SUPERFECT (Qiagen, Inc. Hilden, Germany) and TRANSFECTAM (Promega Biotec, Inc., Madison, WI), as well as other liposomes developed according to procedures standard in the art. In addition, the disclosed nucleic acid or vector can be delivered *in vivo* by electroporation, the technology for which is available from Genetronics, Inc. (San Diego, CA) as well as by means of a SONOPORATION machine (ImaRx Pharmaceutical Corp., Tucson, AZ). Accordingly, and in one aspect, disclosed herein are methods of reducing, repairing, and/or regenerating damaged cardiac tissue due to cardiac dysfunction in a subject in need thereof comprising administering to the subject cardiac induced pluripotent stem cells, wherein the cardiac induced pluripotent stem cells comprises stemness factors, and wherein the stem-cell like factors are inserted into the cardiac or ventricular cells using lipofectamine.

65. The materials may be in solution, suspension (for example, incorporated into microparticles, liposomes, or cells). These may be targeted to a particular cell type via antibodies, receptors, or receptor ligands. The following references are examples of the use of this technology to target specific proteins to tumor tissue (Senter, et al., Bioconjugate Chem., 2:447-451, (1991); Bagshawe, K.D., Br. J. Cancer, 60:275-281, (1989); Bagshawe, et al., Br. J. Cancel, 58:700-703, (1988); Senter, et al., Bioconjugate Chem., 4:3-9, (1993); Battelli, et al., Cancer Immunol. Immunother., 35:421-425, (1992); Pietersz and McKenzie, Immunolog. Reviews, 129:57-80, (1992); and Roffler, et al., Biochem. Pharmacol, 42:2062-2065, (1991)). These techniques can be used for a variety of other specific cell types. Vehicles such as "stealth" and other antibody conjugated liposomes (including lipid mediated drug targeting to colonic carcinoma), receptor mediated targeting of DNA through cell specific ligands, lymphocyte directed tumor targeting, and highly specific therapeutic retroviral targeting of murine glioma cells *in vivo.* The following references are examples of the use of this technology to target specific proteins to tumor tissue (Hughes et al., Cancer Research, 49:6214-6220, (1989); and Litzinger and Huang, Biochimica et Biophysica Acta, 1104:179-187, (1992)). In general, receptors are involved in pathways of endocytosis, either constitutive or ligand induced. These receptors cluster in clathrin-coated pits, enter the cell via clathrin-coated vesicles, pass through an acidified endosome in which the receptors are sorted, and then either recycle to the cell surface, become stored intracellularly, or are degraded in lysosomes. The internalization pathways serve a variety of functions, such as nutrient uptake, removal of activated proteins, clearance of macromolecules, opportunistic entry of viruses and toxins, dissociation and degradation of ligand, and receptor-level regulation. Many receptors follow more than one intracellular pathway, depending on the cell type, receptor concentration, type of ligand, ligand valency, and ligand concentration. Molecular and cellular mechanisms of receptor-mediated endocytosis has been reviewed (Brown and Greene, DNA and Cell Biology 10:6, 399-409 (1991)).

66. Nucleic acids that are delivered to cells which are to be integrated into the host cell genome, typically contain integration sequences. These sequences are often viral related sequences, particularly when viral based systems are used. These viral integration systems can also be incorporated into nucleic acids which are to be delivered using a non-nucleic acid based system of deliver, such as a liposome, so that the nucleic acid contained in the delivery system can be come integrated into the host genome.

67. Other general techniques for integration into the host genome include, for example, systems designed to promote homologous recombination with the host genome. These systems typically rely on sequence flanking the nucleic acid to be expressed that has enough homology with a target sequence within the host cell genome that recombination between the vector nucleic acid and the target nucleic acid takes place, causing the delivered nucleic acid to be integrated into the host genome. These systems and the methods necessary to promote homologous recombination are known to those of skill in the art.

### c) In vivo/ex vivo

68. As described above, the compositions can be administered in a pharmaceutically acceptable carrier and can be delivered to the subject=s cells *in vivo* and/or *ex vivo* by a variety of mechanisms well known in the art (e.g., uptake of naked DNA, liposome fusion, intramuscular injection of DNA via a gene gun, endocytosis and the like).

69. If *ex vivo* methods are employed, cells or tissues can be removed and maintained outside the body according to standard protocols well known in the art. The compositions can be introduced into the cells via any gene transfer mechanism, such as, for example, calcium phosphate mediated gene delivery, electroporation, microinjection or proteoliposomes. The transduced cells can then be infused (e.g., in a pharmaceutically acceptable carrier) or homotopically transplanted back into the subject per standard methods for the cell or tissue type. Standard methods are known for transplantation or infusion of various cells into a subject.

### 2. Homology/identity

70. It is understood that one way to define any known variants and derivatives or those that might arise, of the disclosed genes and proteins herein is through defining the variants and derivatives in terms of homology to specific known sequences. Specifically disclosed are variants of these and other genes and proteins herein disclosed which have at least, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 percent homology to the stated sequence. Those of skill in the art readily understand how to determine the homology of two proteins or nucleic acids, such as genes. For example, the homology can be calculated after aligning the two sequences so that the homology is at its highest level.

71. Another way of calculating homology can be performed by published algorithms. Optimal alignment of sequences for comparison may be conducted by the local homology algorithm of Smith and Waterman Adv. Appl. Math. 2: 482 (1981), by the homology alignment algorithm of Needleman and Wunsch, J. Mol Biol. 48: 443 (1970), by the search for similarity method of Pearson and Lipman, Proc. Natl. Acad. Sci. U.S.A. 85: 2444 (1988), by computerized implementations of these algorithms (GAP, BESTFIT, FASTA, and TFASTA in the Wisconsin Genetics Software Package, Genetics Computer Group, 575 Science Dr., Madison, WI), or by inspection.

### 3. Nucleic acids

72. There are a variety of molecules disclosed herein that are nucleic acid based, including for example the nucleic acids that encode, for example, stemness factor (e.g., Oct3/4, KIf4, Sox2, and c-Myc) as well as any other proteins disclosed herein, as well as various functional nucleic acids. The disclosed nucleic acids are made up of for example, nucleotides, nucleotide analogs, or nucleotide substitutes. Non-limiting examples of these and other molecules are discussed herein. It is understood that for example, when a vector is expressed in a cell, that the expressed mRNA will typically be made up of A, C, G, and U. Likewise, it is understood that if, for example, an antisense molecule is introduced into a cell or cell environment through for example exogenous delivery, it is advantageous that the antisense molecule be made up of nucleotide analogs that reduce the degradation of the antisense molecule in the cellular environment.

### a) Nucleotides and related molecules

73. A nucleotide is a molecule that contains a base moiety, a sugar moiety and a phosphate moiety. Nucleotides can be linked together through their phosphate moieties and sugar moieties creating an internucleoside linkage. The base moiety of a nucleotide can be adenine-9-yl (A), cytosine-1-yl (C), guanine-9-yl (G), uracil-1-yl (U), and thymin-1-yl (T). The sugar moiety of a nucleotide is a ribose or a deoxyribose. The phosphate moiety of a nucleotide is pentavalent phosphate. An non-limiting example of a nucleotide would be 3'-AMP (3'-adenosine monophosphate) or 5'-GMP (5'-guanosine monophosphate).

74. A nucleotide analog is a nucleotide which contains some type of modification to either the base, sugar, or phosphate moieties. Modifications to nucleotides are well known in the art and would include for example, 5-methylcytosine (5-me-C), 5-hydroxymethyl cytosine, xanthine, hypoxanthine, and 2-aminoadenine as well as modifications at the sugar or phosphate moieties.

75. Nucleotide substitutes are molecules having similar functional properties to nucleotides, but which do not contain a phosphate moiety, such as peptide nucleic acid (PNA). Nucleotide substitutes are molecules that will recognize nucleic acids in a Watson-Crick or Hoogsteen manner, but which are linked together through a moiety other than a phosphate moiety. Nucleotide substitutes are able to conform to a double helix type structure when interacting with the appropriate target nucleic acid.

76. It is also possible to link other types of molecules (conjugates) to nucleotides or nucleotide analogs to enhance for example, cellular uptake. Conjugates can be chemically linked to the nucleotide or nucleotide analogs. Such conjugates include but are not limited to lipid moieties such as a cholesterol moiety. (Letsinger et al., Proc. Natl. Acad. Sci. USA, 1989,86, 6553-6556),

77. A Watson-Crick interaction is at least one interaction with the Watson-Crick face of a nucleotide, nucleotide analog, or nucleotide substitute. The Watson-Crick face of a nucleotide, nucleotide analog, or nucleotide substitute includes the C2, N1, and C6 positions of a purine based nucleotide, nucleotide analog, or nucleotide substitute and the C2, N3, C4 positions of a pyrimidine based nucleotide, nucleotide analog, or nucleotide substitute.

78. A Hoogsteen interaction is the interaction that takes place on the Hoogsteen face of a nucleotide or nucleotide analog, which is exposed in the major groove of duplex DNA. The Hoogsteen face includes the N7 position and reactive groups (NH2 or O) at the C6 position of purine nucleotides.

### b) Sequences

79. A variety of sequences are provided herein and these and others can be found in Genbank. Those of skill in the art understand how to resolve sequence discrepancies and differences and to adjust the compositions and methods relating to a particular sequence to other related sequences. Primers and/or probes can be designed for any sequence given the information disclosed herein and known in the art.

### 4. Expression systems

80. The nucleic acids that are delivered to cells typically contain expression controlling systems. For example, the inserted genes in viral and retroviral systems usually contain promoters, and/or enhancers to help control the expression of the desired gene product. A promoter is generally a sequence or sequences of DNA that function when in a relatively fixed location in regard to the transcription start site. A promoter contains core elements required for basic interaction of RNA polymerase and transcription factors, and may contain upstream elements and response elements.

### a) Viral Promoters and Enhancers

81. Preferred promoters controlling transcription from vectors in mammalian host cells may be obtained from various sources, for example, the genomes of viruses such as: polyoma, Simian Virus 40 (SV40), adenovirus, retroviruses, hepatitis-B virus and most preferably cytomegalovirus, or from heterologous mammalian promoters, e.g. beta actin promoter. The early and late promoters of the SV40 virus are conveniently obtained as an SV40 restriction fragment which also contains the SV40 viral origin of replication (Fiers et al., Nature, 273: 113 (1978)). The immediate early promoter of the human cytomegalovirus is conveniently obtained as a *Hin*dIII E restriction fragment (Greenway, P.J. et al., Gene 18: 355-360 (1982)). Of course, promoters from the host cell or related species also are useful herein.

82. Enhancer generally refers to a sequence of DNA that functions at no fixed distance from the transcription start site and can be either 5' (Laimins, L. et al., Proc. Natl. Acad. Sci. 78: 993 (1981)) or 3' (Lusky, M.L., et al., Mol. Cell Bio. 3: 1108 (1983)) to the transcription unit. Furthermore, enhancers can be within an intron (Banerji, J.L. et al., Cell 33: 729 (1983)) as well as within the coding sequence itself (Osborne, T.F., et al., Mol. Cell Bio. 4: 1293 (1984)). They are usually between 10 and 300 bp in length, and they function in cis. Enhancers f unction to increase transcription from nearby promoters. Enhancers also often contain response elements that mediate the regulation of transcription. Promoters can also contain response elements that mediate the regulation of transcription. Enhancers often determine the regulation of expression of a gene. While many enhancer sequences are now known from mammalian genes (globin, elastase, albumin, alpha-fetoprotein and insulin), typically one will use an enhancer from a eukaryotic cell virus for general expression. Preferred examples are the SV40 enhancer on the late side of the replication origin (bp 100-270), the cytomegalovirus early promoter enhancer, the polyoma enhancer on the late side of the replication origin, and adenovirus enhancers.

83. The promotor and/or enhancer may be specifically activated either by light or specific chemical events which trigger their function. Systems can be regulated by reagents such as tetracycline and dexamethasone. There are also ways to enhance viral vector gene expression by exposure to irradiation, such as gamma irradiation, or alkylating chemotherapy drugs.

84. In certain embodiments the promoter and/or enhancer region can act as a constitutive promoter and/or enhancer to maximize expression of the region of the transcription unit to be transcribed. In certain constructs the promoter and/or enhancer region be active in all eukaryotic cell types, even if it is only expressed in a particular type of cell at a particular time. A preferred promoter of this type is the CMV promoter (650 bases). Other preferred promoters are SV40 promoters, cytomegalovirus (full length promoter), and retroviral vector LTR.

85. It has been shown that all specific regulatory elements can be cloned and used to construct expression vectors that are selectively expressed in specific cell types such as melanoma cells. The glial fibrillary acetic protein (GFAP) promoter has been used to selectively express genes in cells of glial origin.

86. Expression vectors used in eukaryotic host cells (yeast, fungi, insect, plant, animal, human or nucleated cells) may also contain sequences necessary for the termination of transcription which may affect mRNA expression. These regions are transcribed as polyadenylated segments in the untranslated portion of the mRNA encoding tissue factor protein. The 3' untranslated regions also include transcription termination sites. It is preferred that the transcription unit also contains a polyadenylation region. One benefit of this region is that it increases the likelihood that the transcribed unit will be processed and transported like mRNA. The identification and use of polyadenylation signals in expression constructs is well established. It is preferred that homologous polyadenylation signals be used in the transgene constructs. In certain transcription units, the polyadenylation region is derived from the SV40 early polyadenylation signal and consists of about 400 bases. It is also preferred that the transcribed units contain other standard sequences alone or in combination with the above sequences improve expression from, or stability of, the construct.

### b) Markers

87. The viral vectors can include nucleic acid sequence encoding a marker product. This marker product is used to determine if the gene has been delivered to the cell and once delivered is being expressed. Preferred marker genes are the *E. Coli* lacZ gene, which encodes ß-galactosidase, and green fluorescent protein.

88. In some embodiments the marker may be a selectable marker. Examples of suitable selectable markers for mammalian cells are dihydrofolate reductase (DHFR), thymidine kinase, neomycin, neomycin analog G418, hydromycin, and puromycin. When such selectable markers are successfully transferred into a mammalian host cell, the transformed mammalian host cell can survive if placed under selective pressure. There are two widely used distinct categories of selective regimes. The first category is based on a cell's metabolism and the use of a mutant cell line which lacks the ability to grow independent of a supplemented media. Two examples are: CHO DHFR- cells and mouse LTK- cells. These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented media. An alternative to supplementing the media is to introduce an intact DHFR or TK gene into cells lacking the respective genes, thus altering their growth requirements. Individual cells which were not transformed with the DHFR or TK gene will not be capable of survival in non-supplemented media.

89. The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, (Southern P. and Berg, P., J. Molec. Appl. Genet. 1: 327 (1982)), mycophenolic acid, (Mulligan, R.C. and Berg, P. Science 209: 1422 (1980)) or hygromycin, (Sugden, B. et al., Mol. Cell. Biol. 5: 410-413 (1985)). The three examples employ bacterial genes under eukaryotic control to convey resistance to the appropriate drug G418 or neomycin (geneticin), xgpt (mycophenolic acid) or hygromycin, respectively. Others include the neomycin analog G418 and puramycin.

### 5. Pharmaceutical carriers/Delivery of pharmaceutical products

90. As described herein, the compositions can also be administered *in vivo* in a pharmaceutically acceptable carrier. By "pharmaceutically acceptable" is meant a material that is not biologically or otherwise undesirable, i.e., the material may be administered to a subject, along with the nucleic acid or vector, without causing any undesirable biological effects or interacting in a deleterious manner with any of the other components of the pharmaceutical composition in which it is contained. The carrier would naturally be selected to minimize any degradation of the active ingredient and to minimize any adverse side effects in the subject, as would be well known to one of skill in the art.

91. The compositions may be administered orally, parenterally (e.g., intravenously), by intramuscular injection, by intraperitoneal injection, intramyocardial injection, transdermally, extracorporeally, topically or the like, including topical intranasal administration or administration by inhalant. As used herein, "topical intranasal administration" means delivery of the compositions into the nose and nasal passages through one or both of the nares and can comprise delivery by a spraying mechanism or droplet mechanism, or through aerosolization of the nucleic acid or vector. Administration of the compositions by inhalant can be through the nose or mouth via delivery by a spraying or droplet mechanism. Delivery can also be directly to any area of the respiratory system (e.g., lungs) via intubation. The exact amount of the compositions required will vary from subject to subject, depending on the species, age, weight and general condition of the subject, the severity of the allergic disorder being treated, the particular nucleic acid or vector used, its mode of administration and the like. Thus, it is not possible to specify an exact amount for every composition. However, an appropriate amount can be determined by one of ordinary skill in the art using only routine experimentation given the teachings herein. For example, the cardiac induced pluripotent stems cells disclosed herein can be administered by parenteral injection to the site of tissue damage, for example the peri-infarct region.

92. Accordingly, disclosed herein are methods of reducing, repairing, and/or regenerating damaged cardiac tissue or reducing infarct size in a subject in need thereof comprising administering to the subject cardiac induced pluripotent stem cells (iPS), wherein in the iPS are administered via parenteral injection such as an intra-myocardial injection to the peri-infarct region.

93. Parenteral administration of the composition, if used, is generally characterized by injection. Injectables can be prepared in conventional forms, either as liquid solutions or suspensions, solid forms suitable for solution of suspension in liquid prior to injection, or as emulsions. A more recently revised approach for parenteral administration involves use of a slow release or sustained release system such that a constant dosage is maintained.

94. The materials may be in solution, suspension (for example, incorporated into microparticles, liposomes, or cells). These may be targeted to a particular cell type via antibodies, receptors, or receptor ligands. The following references are examples of the use of this technology to target specific proteins to tumor tissue (Senter, et al., Bioconjugate Chem., 2:447-451, (1991); Bagshawe, K.D., Br. J. Cancer, 60:275-281, (1989); Bagshawe, et al., Br. J. Cancer, 58:700-703, (1988); Senter, et al., Bioconjugate Chem., 4:3-9, (1993); Battelli, et al., Cancer Immunol. Immunother., 35:421-425, (1992); Pietersz and McKenzie, Immunolog. Reviews, 129:57-80, (1992); and Roffler, et al., Biochem. Pharmacol, 42:2062-2065, (1991)). Vehicles such as "stealth" and other antibody conjugated liposomes (including lipid mediated drug targeting to colonic carcinoma), receptor mediated targeting of DNA through cell specific ligands, lymphocyte directed tumor targeting, and highly specific therapeutic retroviral targeting of murine glioma cells *in vivo.* The following references are examples of the use of this technology to target specific proteins to tumor tissue (Hughes et al., Cancer Research, 49:6214-6220, (1989); and Litzinger and Huang, Biochimica et Biophysica Acta, 1104:179-187, (1992)). In general, receptors are involved in pathways of endocytosis, either constitutive or ligand induced. These receptors cluster in clathrin-coated pits, enter the cell via clathrin-coated vesicles, pass through an acidified endosome in which the receptors are sorted, and then either recycle to the cell surface, become stored intracellularly, or are degraded in lysosomes. The internalization pathways serve a variety of functions, such as nutrient uptake, removal of activated proteins, clearance of macromolecules, opportunistic entry of viruses and toxins, dissociation and degradation of ligand, and receptor-level regulation. Many receptors follow more than one intracellular pathway, depending on the cell type, receptor concentration, type of ligand, ligand valency, and ligand concentration. Molecular and cellular mechanisms of receptor-mediated endocytosis has been reviewed (Brown and Greene, DNA and Cell Biology 10:6, 399-409 (1991)).

### a) Pharmaceutically Acceptable Carriers

95. The compositions, including antibodies, can be used therapeutically in combination with a pharmaceutically acceptable carrier.

96. Suitable carriers and their formulations are described in Remington: The Science and Practice of Pharmacy (19th ed.) ed. A.R. Gennaro, Mack Publishing Company, Easton, PA 1995. Typically, an appropriate amount of a pharmaceutically-acceptable salt is used in the formulation to render the formulation isotonic. Examples of the pharmaceutically-acceptable carrier include, but are not limited to, saline, Ringer's solution and dextrose solution. The pH of the solution is preferably from about 5 to about 8, and more preferably from about 7 to about 7.5. Further carriers include sustained release preparations such as semipermeable matrices of solid hydrophobic polymers containing the antibody, which matrices are in the form of shaped articles, e.g., films, liposomes or microparticles. It will be apparent to those persons skilled in the art that certain carriers may be more preferable depending upon, for instance, the route of administration and concentration of composition being administered.

97. Pharmaceutical carriers are known to those skilled in the art. These most typically would be standard carriers for administration of drugs to humans, including solutions such as sterile water, saline, and buffered solutions at physiological pH. The compositions can be administered intramuscularly or subcutaneously. Other compounds will be administered according to standard procedures used by those skilled in the art.

98. Pharmaceutical compositions may include carriers, thickeners, diluents, buffers, preservatives, surface active agents and the like in addition to the molecule of choice. Pharmaceutical compositions may also include one or more active ingredients such as antimicrobial agents, anti-inflammatory agents, anesthetics, and the like.

99. The pharmaceutical composition may be administered in a number of ways depending on whether local or systemic treatment is desired, and on the area to be treated. Administration may be topically (including ophthalmically, vaginally, rectally, intranasally), orally, by inhalation, or parenterally, for example by intravenous drip, subcutaneous, intraperitoneal or intramuscular injection. The disclosed antibodies can be administered intravenously, intraperitoneally, intramuscularly, subcutaneously, intracavity, or transdermally.

100. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

101. Formulations for topical administration may include ointments, lotions, creams, gels, drops, suppositories, sprays, liquids and powders. Conventional pharmaceutical carriers, aqueous, powder or oily bases, thickeners and the like may be necessary or desirable.

102. Compositions for oral administration include powders or granules, suspensions or solutions in water or non-aqueous media, capsules, sachets, or tablets. Thickeners, flavorings, diluents, emulsifiers, dispersing aids or binders may be desirable..

103. Some of the compositions may potentially be administered as a pharmaceutically acceptable acid- or base- addition salt, formed by reaction with inorganic acids such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, maleic acid, and fumaric acid, or by reaction with an inorganic base such as sodium hydroxide, ammonium hydroxide, potassium hydroxide, and organic bases such as mono-, di-, trialkyl and aryl amines and substituted ethanolamines.

### b) Therapeutic Uses

104. Effective dosages and schedules for administering the compositions may be determined empirically, and making such determinations is within the skill in the art. The dosage ranges for the administration of the compositions are those large enough to produce the desired effect in which the symptoms/disorder are/is effected. The dosage should not be so large as to cause adverse side effects, such as unwanted cross-reactions, anaphylactic reactions, and the like. Generally, the dosage will vary with the age, condition, sex and extent of the disease in the patient, route of administration, or whether other drugs are included in the regimen, and can be determined by one of skill in the art. The dosage can be adjusted by the individual physician in the event of any contraindications. Dosage can vary, and can be administered in one or more dose administrations daily, for one or several days. Guidance can be found in the literature for appropriate dosages for given classes of pharmaceutical products. For example, guidance in selecting appropriate doses for antibodies can be found in the literature on therapeutic uses of antibodies, e.g., Handbook of Monoclonal Antibodies, Ferrone et al., eds., Noges Publications, Park Ridge, N.J., (1985) ch. 22 and pp. 303-357; Smith et al., Antibodies in Human Diagnosis and Therapy, Haber et al., eds., Raven Press, New York (1977) pp. 365-389. A typical dosage of the cardiac induced pluripotent stem cells can be between 50,000 and 50,000,000 iPS, depending on the factors mentioned above. For example, the administered dose can be 50,000; 55,000; 60,000; 65,000; 70,000; 75,000; 80,000; 90,000; 100,000; 110,000; 120,000; 130,000; 140,000; 150,000; 200,000; 300,000; 400,000; 500,000; 600,000; 700,000; 800,000; 900,000; 1,000,000; 10,000,000; or 50,000;000 cells or any amount in between. Thus for example the amount of cells administered can be 100,000 to 50,000,000 cells.

105. It is further understood that the administered dose can be applied in one or multiple injections. Specifically contemplated are instances wherein 1, 2, 3, 4, 56, 7, 8, 9, or 10 injections to the subject are made to deliver the total dosage, the amount of cells delivered for each injection appropriately reduced such that when the amount of cells for each injection is added, the total dosage is reached. For example, a dosage of 50,000 cells administered in two injections would be two 25,000 cell injections. Thus, for example, disclosed herein are methods of reducing, repairing, or regenerating cardiac tissue damage in a subject in need thereof comprising administering to the subject iPS, wherein the between 50,000 and 500,000 iPS are administered to the subject. Also disclosed are methods wherein the subject receives the dose of 50,000 to 500,000 iPS in two equal injections (e.g., two equal intramyocardial injections).

106. Following administration of a disclosed composition, such as an antibody, for treating, inhibiting, or preventing an ischemia/reperfusion injury, the efficacy of the therapeutic antibody can be assessed in various ways well known to the skilled practitioner.

### D. Examples

107. The following examples are put forth so as to provide those of ordinary skill in the art with a complete disclosure and description of how the compounds, compositions, articles, devices and/or methods claimed herein are made and evaluated, and are intended to be purely exemplary and are not intended to limit the disclosure. Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric.

### 1. Example 1

108. Cardiac myocyte differentiation reported thus far is from iPS cells generated from mice and human fibroblasts. The present invention utilized cardiac cells transfected with stem-cell like factors. The example below uses rat cardiac cells to generate iPS cells, however the disclosure herein may be used with any other cardiac and ventricular cells, such as human or rodent cardiac cells. Other exemplary cells include primary human cardiac myocytes or human fetal cells available commercially, such as cells from ATCC (Manassas, VA).

### a) Cell Line Maintenance

109. Rat cardiomyoblast H9c2 cells, originally isolated from embryonic rat heart ventricular tissue (Hescheler, et al., Morphological, biochemical, and electrophysiological characterization of a clonal cell (H9c2) line from rat heart. Circ Res 1991;69:1476-1486; Kimes & Brandt, Properties of a clonal muscle cell line from rat heart. Exp Cell Res 1976;98:367-381), was ordered from American type cell culture (ATCC) and maintained in DMEM. H9c2 cells and undifferentiated mouse CGR8 ES cells expressing red fluorescence protein (RFP) were maintained on gelatin plates as previously published (Singla et al., Factors Released from Embryonic Stem Cells inhibit Apoptosis in H9c2 cells through Pl-3kinase/Akt but not ERK pathway. Am J Physiol Heart Circ Phisiol;295:H907-H913).

### b) Generation of Transduced Vectors and iPS cells

110. The cDNAs coding Oct3/4, KIf4, Sox2 and c-Myc were generated by PCR cloning using high fidelity DNA polymerase and PCR primers (Oct3/4 5'-CACC ATGGCTGGACACCTGGCTTC-3' and 5'-GTTTGAATGCATGGGAGA-3', Klf4: 5'-ATGGCTGTCAGCGACGCTCT-3' and 5'-AAAGTGCCTCTTCATGTGTAAG-3', Sox2: 5'-ATGTATAACATGATGGAGACG-3' and TCACATGTGCGAC AGGGGCAGTGT-3', c-Myc: 5'-TTCACCATGCCCCTCAACGTGAACTT-3' and 5'-TTTATGCACCAGAGTTCG-3') and were then cloned into vector pCR2.1 (Invitrogen, Carlsbad, CA). The cDNA sequences for KIf4, Sox2 and Oct3/4 were linked through the self-cleaving 2A sequence of foot-and-mouth disease virus (5'-AAAATTGTCGCTCCTGTCAAACAAACTCT TAACTTTGATTTACTCAAACTGGCTGGGATGTAGAAAGCAATCCAGGTC CA-3'). The sense and antisense oligonucleotides containing 2A sequence was ligated into vector pBluscriptll SK(-) (pBS, Stratagene, La Jolla, CA), and the generated the vector pBS-2A required for further cloning. Next, Oct3/4 and KIf4 cDNAs were independently inserted into vector pBS-2A, which generated pBS-Oct3/4-2A and pBS-KIf4-2A. Furthermore, KIf4-2A sequence was isolated, and then inserted into pBS-Oct-3/4-2A in the same reading frame to form pBS-Oct-3/4-2A-KIf4-2A, which was then fused to the Sox2 cDNA with a stop codon in the same reading frame to form pBS-Oct-3/4-2A-KIf4-2A-Sox2. The cDNA fragment of Oct3/4-2A-KIf4-2A-Sox2 was removed from the vector and cloned into the EcoRI sites of the expression vector pCX-EGFP (obtained from Dr. Masaru Okabe, Japan). For the fourth gene c-MYC cloning, EGFP present in the Oct3/4-2A-KIf4-2A-Sox2-pCX-EGFP was replaced with c-Myc cDNA. The end product generated was an expression vector containing the four genes as mentioned above.

111. The H9c2 cells were cultured in 6-well plates at a density of 20,000 cells per well in DMEM containing 10% FBS. Cells were grown to 80% confluency and then transduced with the expression plasmids containing Oct-3/4, Sox2, KIf4 and c-Myc, using Lipofectamin 2000 (Invitrogen) concurring to the manufacturer's instructions. Subsequent transductions were performed on day 3 and 5, cells were incubated for seven days, and media were changed every 48 hours.

112. Transduced H9c2 cells converted into iPS cells were trypsinized, plated on gelatin-coated plates, and cultured in a stem cell growth medium as mentioned above. Subsequently, these cells start forming ES cell-like colonies after three weeks. The ES cell-like colonies were extracted and cultured further on gelatin-coated plates in the ES cell culture medium and conditioned medium (CM) prepared from mouse embryonic fibroblasts (MEF) cells (50:50). Furthermore, iPS and H9c2 cells were transfected with a RFP expression vector, and stable RFP cell clones were selected with antibiotics.

113. Western blotting was carried out as published (Singla & Sun, Transforming growth factor-beta2 enhances differentiation of cardiac myocytes from embryonic stem cells. Biochem Biophys Res Commun 2005;332:135-141) before to verify the expression of transduced stemness factors (4F) in the generated cardiac iPS cells from H9c2 cells. In brief, iPS or H9c2 cells (control) were lysed, centrifuged, and supernatant was isolated. Proteins were run on SDS-Page (10% polyacrylamide gel) and transferred onto PVDF membranes. After washings, incubation was performed with primary antibodies (anti-c-Myc, anti-Sox2, anti-Klf4, and anti-Oct3/4) followed by horseradish peroxidase-conjugated secondary antibodies at room temperature. After incubations, reaction was considerably matured with enhanced chemiluminescence kit (GE Life Sciences) and captured on X-ray film (Sigma, Kodak).

### c) Determination of Pluripotency

114. Generated iPS cells, ES cells (positive control) or H9c2 cells (negative control) were washed 2-3 times with PBS for 5-7 min. After washings, cells were stained with an alkaline phosphatase kit (Chemicon International, USA) as per manufacturer's specific recommendations. Then, cells were examined through a bright field converted microscope, and pictures were brilliantly captured.

115. For immunostaining, cells were fixed and permeabilized with methanol:acetone (7:3) for 20 min at -20°C, and incubated with primary antibody against Oct-3/4, a pluripotent marker present on the ES cells. Following three washings, cells were incubated with 2° antibody Alexa Fluor 568-conjugated with IgG (Molecular Probe). The nuclei were identified with DAPI present in the mounting medium (Vector Laboratories). Cells were examined with a Leica TSC SP2 laser scanning confocal microscope.

116. Next, the embryoid bodies (EBs) were generated, which contain cells from all three germ layers as reported, using standard hanging drop method from iPS or ES cells (Singla, et al., Transplantation of embryonic stem cells into the infarcted mouse heart: formation of multiple cell types. J Mol Cell Cardiol 2006;40:195-200; Wiles & Keller, Multiple hematopoietic lineages develop from embryonic stem (ES) cells in culture. Development 1991;111:259-267). In brief, iPS or ES cells were cultured without LIF at a concentration of 2.5x10⁴ cells/ml. Using the hanging drop method, 30 µl cell suspension containing 600-750 cells were developed into hanging drops in the cell culture plate for two days and then plated into in a suspension culture for an additional two days. Then, each EB was extracted and placed on gelatin coated plates for up to 17 days. The generated EBs from both groups were compared to determine pluripotency as well as used for the differentiation into cardiac myocytes.

### d) Cell transplantation in the Infarcted Mouse Heart

117. Animal protocols used in the current study were approved by the University of Central Florida animal care committee. MI was performed as reported (Singla, et al., Transplantation of embryonic stem cells into the infarcted mouse heart: formation of multiple cell types. J Mol Cell Cardiol 2006;40:195-200; Kumar, et al., Distinct mouse coronary anatomy and myocardial infarction consequent to ligation. Coron Artery Dis 2005;16:41-44). In brief, male and female 8- to 10 week old (C57BL/6) mice were anesthetized with isoflurane, left descending coronary artery was identified and ligated with 7-0 suture as performed in the laboratory routinely. Animals demonstrating discoloration below the ligation were considered as infarcted animals and used for cell or media transplantation. Within a few minutes following ligation, iPS cells (n=8), ES cells (n=8), or H9c2 cells (n=8) were transplanted by two intra-myocardial injections of 10 µl each containing 25,000 cells/injection or cell culture medium (n=8), in the peri-infarct region. After 2 weeks, heart function was determined using echocardiography as published (Singla, et al., Transplantation of embryonic stem cells into the infarcted mouse heart: formation of multiple cell types. J Mol Cell Cardiol 2006;40:195-200; Wiles & Keller, Multiple hematopoietic lineages develop from embryonic stem (ES) cells in culture. Development 1991;111:259-267; Kumar, et al., Distinct mouse coronary anatomy and myocardial infarction consequent to ligation. Coron Artery Dis 2005;16:41-44), and animals were sacrificed using pentobarbital (80 mg/kg, IP) injection followed by cervical dislocation. Hearts were isolated, and fixed in buffered formalin (10%) to perform further analysis.

### e) Preparation of Tissue Sections and Histopathology

118. Paraffin embedded heart tissue was cut into 5 µm thick sections, deparaffinized, and rehydration was performed as reported previously (Singla, et al., Transplanted embryonic stem cells following mouse myocardial infarction inhibit apoptosis and cardiac remodeling. Am J Physiol Heart Circ Physiol 2007;293:H1308-H1314). To determine cell morphology and fibrosis, sections from all heart groups were then stained with hematoxylin and eosin, and Masson's Trichrome, respectively. Cardiac fibrosis, predominantly present in the left ventricle (LV), was observed and quantified by measuring the total blue area per mm² with image J NIH program in the Masson's Trichrome stained sections.

### f) Identification of Differentiated Cardiac myocytes

119. Immunostainings on EBs or trypsinized beating area of the EBs were performed as reported previously (Singla & Sun, Transforming growth factor-beta2 enhances differentiation of cardiac myocytes from embryonic stem cells. Biochem Biophys Res Commun 2005;332:135-141). In brief, cells were fixed and blocked with 10% normal goat serum (NGS) followed by incubation with primary antibodies, anti-sarcomeric cardiac *a*-actin (1:100 sigma) or myosin heavy chain antibody (MHC), MF-20 (1:100, hybridoma technologies) at room temperature (RT) for 1 hour. After incubation, cells were treated with secondary antibodies, goat anti-mouse IgM or IgG (1:75, Invitrogen). Finally, sections were washed with PBS, air dried, and mounted with antifade Vectashield mounting medium containing 4,6-diamidino-2-phenylindole (DAPI; Vector Laboratories) to stain all cellular nuclei. H9c2 cells were used as negative controls and ES cells were positive controls. Sections were examined and imaged with confocal microscope as mentioned above.

120. Heart sections were deparaffined, dehydrated and incubated with 10% NGS to block non-specific bindings. In brief, sections were incubated with primary antibody anti-RFP to detect donor cells; iPS cells, ES cells or H9c2 cells. Heart sections were co-labeled with primary antibody; mouse mAb anti sarcomeric *a*-actin (Sigma) for cardiomyocytes followed by incubations with secondary antibodies: anti-mouse or anti-rabbit Alexa 568, anti-mouse IgG conjugated with FITC, or rhodamine (M.O.M. kit, Vector Laboratories). Each set of staining includes control sections by omitting primary or secondary antibody. Sections were mounted with antifade containing DAPI to stain all nuclei, and were visualized under confocal microscope.

### g) Apoptosis

121. Heart sections were deparaffinized, dehydrated and exposed to proteinase K for 20 min. at room temperature for permeabilization. A commercially available apoptotic cell death detection kit was obtained from Roche Applied Bio Sciences, USA. Apoptotic staining was performed following strict instructions as explained in the flyer provided with the kit to detect apoptotic nuclei in the heart sections. The amount of apoptosis was determined by counting red apoptotic nuclei in the 1-2 heart sections from 6-8 different hearts as reported previously (Singla, et al., Transplanted embryonic stem cells following mouse myocardial infarction inhibit apoptosis and cardiac remodeling. Am J Physiol Heart Circ Physiol 2007;293:H1308-H1314). Apoptotic nuclei counted per section were converted into percent positive by counting the total red stained TUNEL positive nuclei divided by total blue stained DAPI positive nuclei in 4-6 randomly selected fields in the infarct and border zone area at 20x.

122. For caspase 3 staining, sections were co-labeled with primary antibody anti-caspase 3 rabbit polyclonal (1:50 dilution, Santa Cruz Biotechnology and cell signaling) and sarcomeric cardiac *a*-actin mouse monoclonal antibody (1:20; Sigma) for 1 hour at 37 °C in a humidified chamber. Three washings were performed and sections were incubated with anti-rabbit Alexa 635 or anti-mouse Alexa 488 secondary antibodies (Molecular Probes). Sections were mounted with antifade medium containing DAPI (Vector Laboratories) to stain nuclei. Sections were examined with a confocal microscope.

123. Caspase-3 activity was performed using Bio-vision colorimetric assay as reported previously (Singla DK, Singla RD, McDonald DE. Factors Released from Embryonic Stem Cells inhibit Apoptosis in H9c2 cells through Pl-3kinase/Akt but not ERK pathway. Am J Physiol Heart Circ Physiol;295:H907-H913; Fatma S, Selby DE, Singla RD, Singla DK. Factors Released From Embryonic Stem Cells Stimulate c-kit-FlK-1+ve Progenitor Cells and Enhance Neovascularization. Antioxid Redox Signal 2010; [Epub ahead of print]).

124. Group data was presented as means±SE. One way ANOVA followed by Tukey test to assess the significant difference between groups, p<0.05.

### 2. Example 2: Cardiac iPS cells were analyzed to determine their pluripotency.

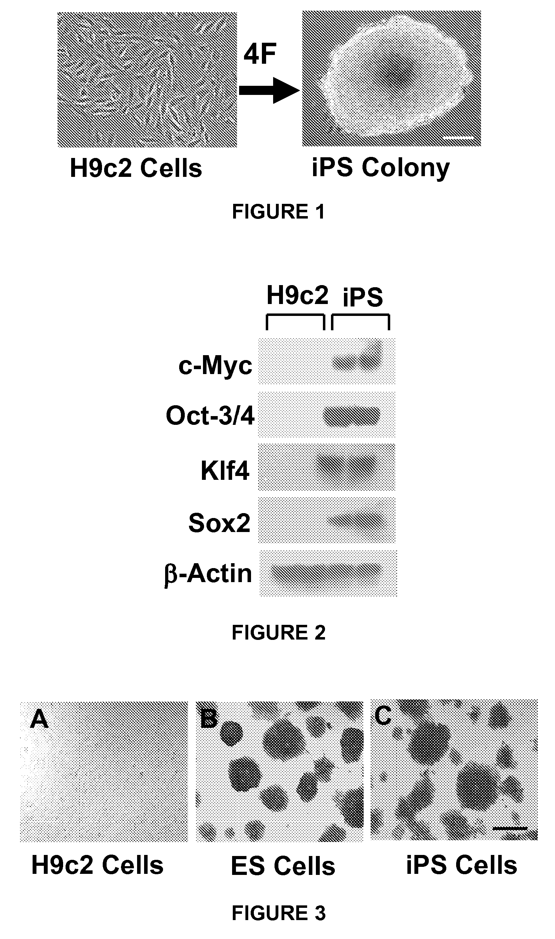

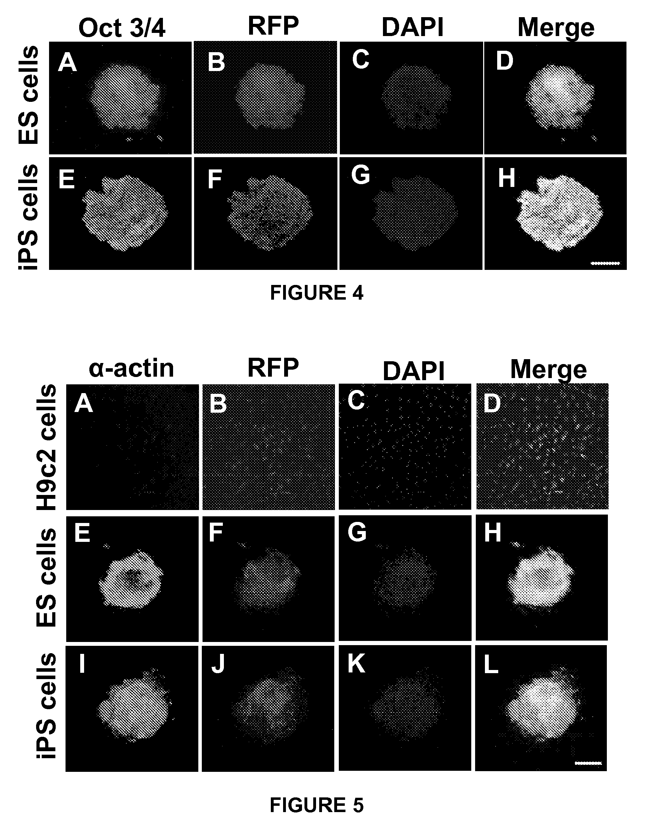

125. Figures 1(a) and (b) show H9c2 cells are elongated, whereas following transduction with 4F expression vector, these cells start appearing morphologically, seen in Figure 1(b), distinct compared with untransduced H9c2 cells, seen in Figure 1(a). Moreover, transduced H9c2 cell morphology appeared to be similar to growing mouse ES cells (Singla, et al., wnt3a but not wnt11 supports self-renewal of embryonic stem cells. Biochem Biophys Res Commun 2006;345:789-795). The efficiency of cluster formation varied from .008-.01% in H9c2 cells following transduction. Subsequently, growing clones were monitored, extracted, and maintained in cell culture medium containing MEF conditioned medium. To determine whether these four factors are present in the generated iPS cells, the western blot analysis data shows expression for these four sternness factors in the iPS cells, whereas no such factor was present in the H9c2 cells, seen in Figure 2. Next, cardiac iPS cells were analyzed to determine whether generated iPS cells also express pluripotency markers such as alkaline phosphatase and Oct3/4. As expected, iPS cells expressed alkaline phosphatase in 100% of growing cells, whereas similar expression was observed in ES cells used as a positive control, seen in Figures 3(b) and (c). In contrast, H9c2 cells were negative for alkaline phosphatase staining, seen in Figure 3(a). Moreover, growing iPS cells were positive for the undifferentiated pluripotent marker Oct3/4, which was compared with ES cells as a positive control, seen in Figures 4(a)-(d). These data suggest that generated iPS cells are reprogrammed, express pluripotent markers, and comparable to ES cells.

### 3. Example 3: Cardiac Myocyte Differentiation From iPS Cells in Cell Culture

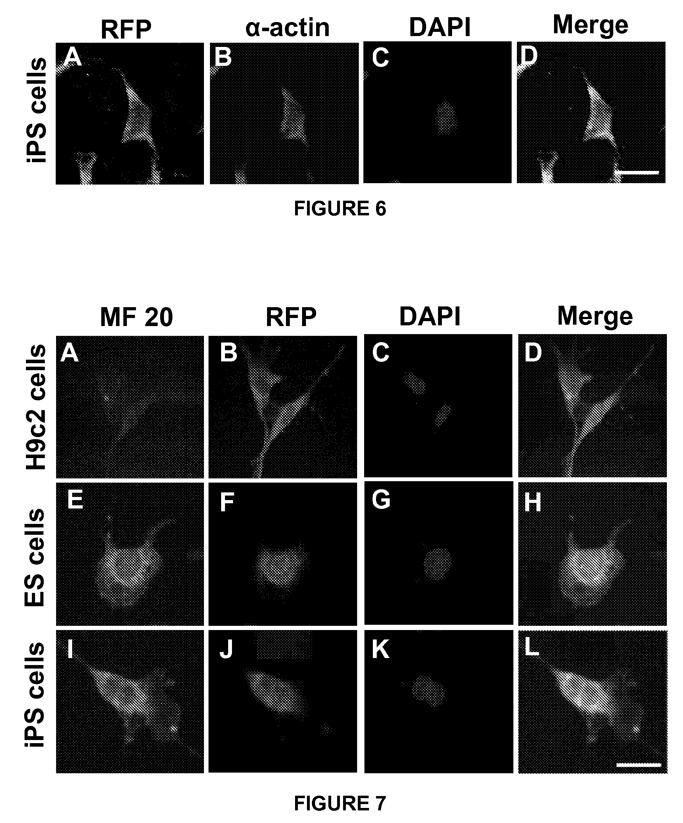

126. To test whether cardiac iPS cells have the potential to generate EBs which contain cells from all 3 germ layers as observed with ES cells, EBs were generated using standard hanging drop method. iPS cell-generated EBs were morphologically and functionally similar to ES cell derived EBs (data not shown). Next, EBs potential to generate cardiac myocytes at day 11 was determined. 30% of spontaneously beating EBs were observed generated from iPS cells compared with 27% from ES cells, suggesting the presence of cardiac myocytes with similar potential in both cell types (data not shown). Furthermore, double immunolabeling for the cardiac-specific marker, sarcomeric *a*-actin, and reporter protein RFP were performed to determine the presence of cardiac myocytes. Certain EBs generated from iPS cells or ES cells were highly positive for sarcomeric *a-*actin demonstrating the presence of cardiac myocytes (sarcomeric *a*-actin, seen in Figures 5(a), (e) and (i)) and reporter protein RFP (Figure 5(b), (f) and (j)). DAP1 stained total nuclei (Figure 5(c), (g) and (k)), and merged images confirm cardiac myocytes differentiation (Figure 5(d), (h) and (l)). Next, the beating EBs were trypsinized and stained with sarcomeric *a*-actin as shown in Figures 6(a)-(d). Moreover, digested EBs were also stained with another cardiac myocyte specific marker, MHC antibody, MF-20, in iPS and ES-EBs (Figure 7(i)-(l) and (e)-(h)) whereas H9c2 cells were negative for MF-20 (Figure 7(a)-(d)). The quantitative data suggest that there were 25% differentiated cardiac myocytes in the EBs generated from both iPS and ES cells compared with 0% in H9c2 cells. Therefore, it is suggested that the cardiac iPS cell line has the potential to differentiate into cardiac myocytes in cell culture.

### 4. Example 4: Transplanted iPS Cells Differentiate into Cardiac Myocytes and Form Gap Junctions

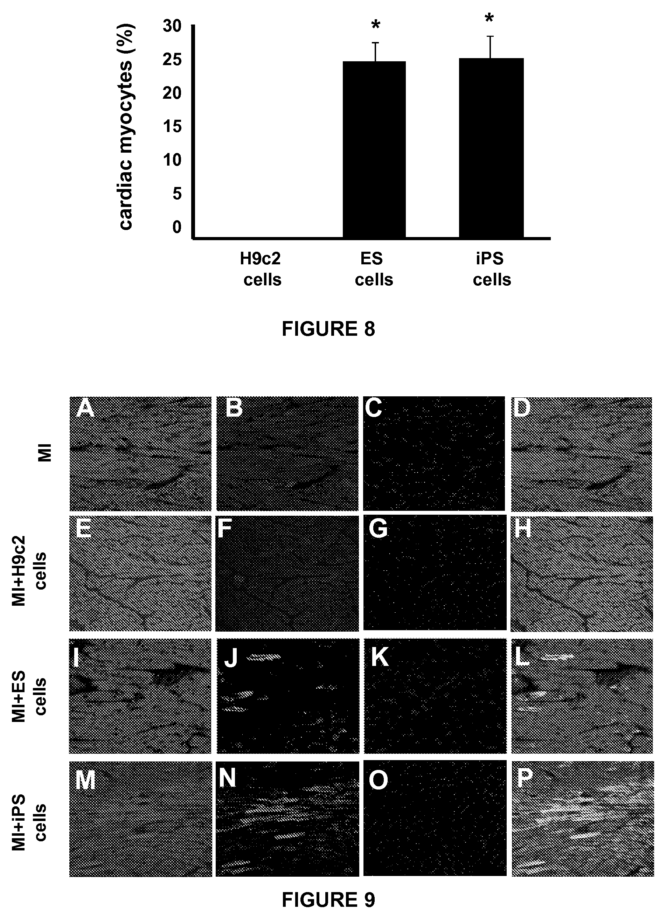

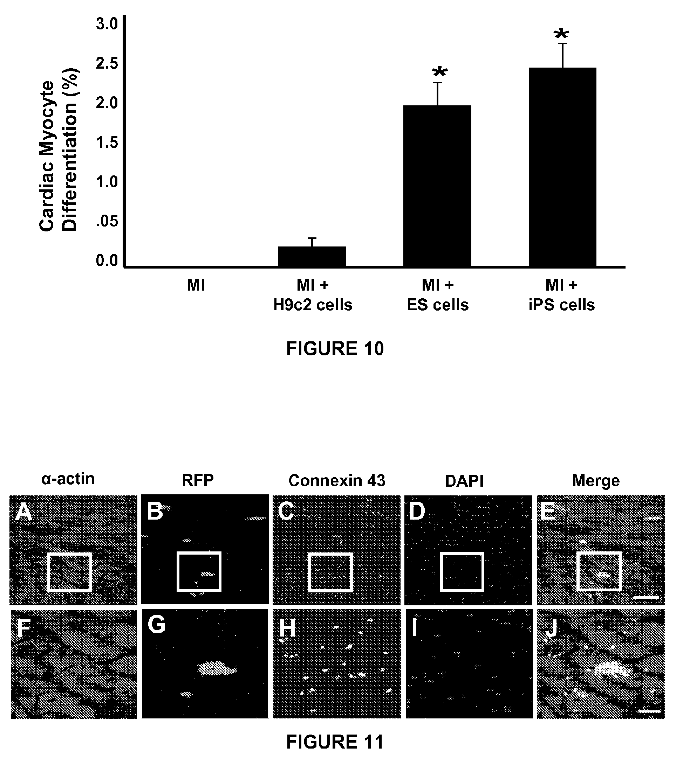

127. Heart sections stained with anti-RFP antibody demonstrated that the transplanted iPS cells were present in the peri-infarct and infarct regions of the myocardium in C57BL/6 mice (Figure 9(n)) whereas there were no stained cells in H9c2 cell transplanted hearts. Next, MI+ES cell transplanted hearts stained with anti-RFP antibody demonstrated patchy areas of engrafted cells in the peri-infarct and infarct region of the heart (Figure 9(j)). To confirm whether transplanted cells differentiated into cardiac myocytes, all four groups were co-labeled with sarcomeric *a*-actin antibody, suggesting transplanted stem cells differentiated into new cardiac myocytes (Figure 9(i)-(p)). However, newly generated cardiac myocytes were rarely found in the H9c2 cell transplanted group. Quantitative data shows that there were 1.92±0.3% newly formed cardiac myocytes in ES cell transplanted hearts compared with 2.37±0.3% in iPS cells (Figure 10). The percent positive differentiated cardiac myocytes in iPS and ES groups were significantly (p<0.05) different compared with H9c2 cells and MI groups. However, there was no statistical difference between iPS and ES cell groups (Figure 10). Next, to confirm whether newly generated cardiac myocytes demonstrate electrical coupling in the native myocardium, heart sections were triple immunolabeled; RFP to detect donor iPS cells, sarcomeric *a*-actin, to detect cardiac myocytes and connexin-43, to identify gap junctions. Figures 11(a)-(l), shows newly generated cardiac myocytes form gap junctions, were electrically coupled, and integrated into the myocardium.

### 5. Example 5: Transplanted iPS Cells Inhibit Apoptosis

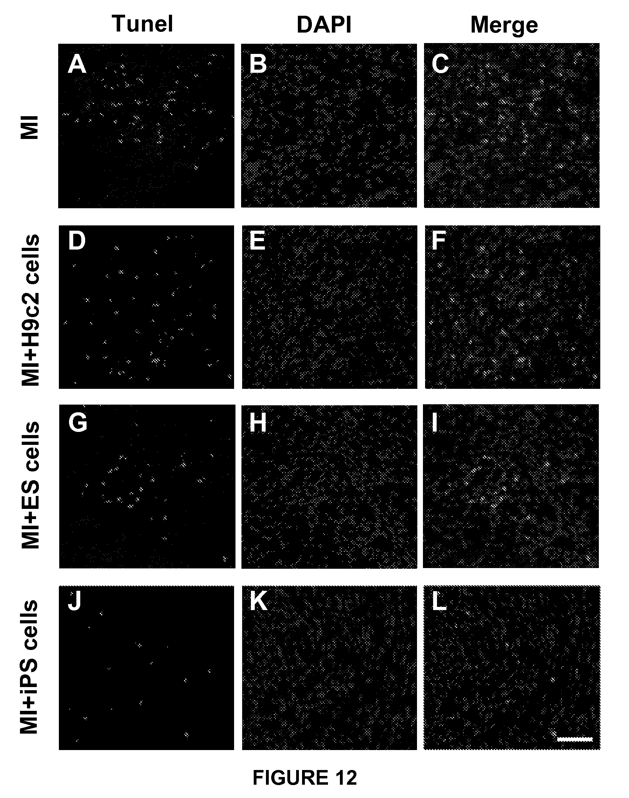

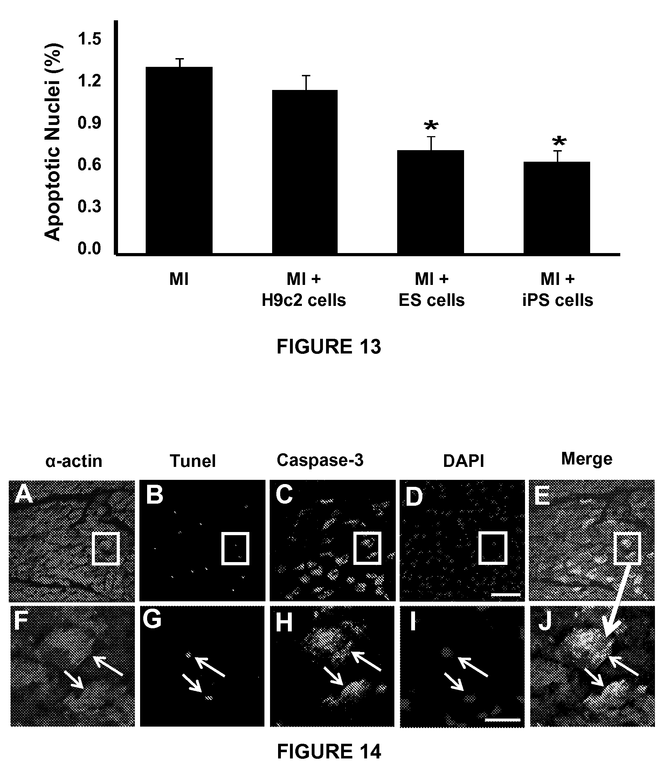

128. Effects of transplanted iPS cells on cardiac apoptosis were determined by TUNEL staining, caspase-3 immunostaining, and caspase-3 activity. TUNEL staining data shows wide spread red apoptotic nuclei in MI and MI+H9c2 cell groups (Figures 12(a) and (d)) compared with the reduced amount of red apoptotic nuclei in MI+iPS or ES cell groups (Figure 12(g) and (j)). DAPI stained total nuclei (Figure 12(b), (e), (h) and (k)) and merged images are visible in Figures 12(c), (f), (i) and (l). Quantitative TUNEL apoptotic nuclei shows transplanted iPS cells significantly (p<0.05) reduce apoptotic nuclei (0.6±.07%) compared with MI or MI+H9c2 cell groups (MI; 1.2±0.1, MI±H9c2 cells; 1+0.2% apoptotic nuclei/section, Figure 13). Moreover, transplanted ES cells reduced apoptosis comparable with the iPS cell group. TUNEL stained heart sections were also combined with caspase-3 staining to confirm cell death is apoptotic in nature. Furthermore, some of the sections were also co-stained with a third antibody, sarcomeric *a*-actin, to confirm apoptosis occurs in the cardiac myocytes. Figures 14(a)-(j), confirm apoptotic nuclei were stained with TUNEL, co-labeled with caspase-3 antibody, and also positive with sarcomeric *a*-actin, suggesting apoptosis occurs in cardiac myocytes. Next, caspase-3 activity was measured, which is considered a hallmark of apoptosis. Our data shows that following iPS or ES cell transplantation, caspase-3 activity was significantly (p<0.05) reduced compared with MI, whereas H9c2 cells demonstrated no decrease in caspase-3 activity. Moreover, caspase-3 activity data corroborate with the TUNEL apoptotic nuclei staining confirming apoptosis in the heart.

### 6. Example 6: Transplanted iPS cells Inhibit Fibrosis

129. Histological examinations were performed to determine whether transplanted iPS or ES cells inhibit adverse cardiac remodeling, LV remodeling was determined by measuring total interstitial fibrotic area (in mm²) in all mice groups transplanted with or without stem cells. Heart sections obtained from infarcted hearts transplanted with iPS and ES cells show significant (p<0.05) decrease in fibrosis (MI+iPS cells: 0.25±0.09 and MI+ES cells: 0.21±.05 mm2) compared with large fibrotic areas in MI and H9c2 cell transplanted hearts (MI.0.94+0.03 and MI+H9c2 cells: 0.9±07 mm2).

### 7. Example 7: Teratoma Formation

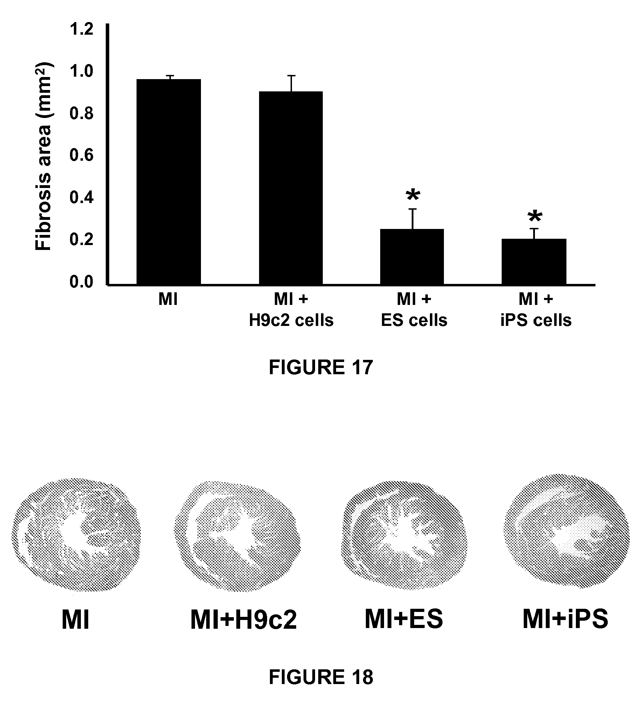

130. All animals transplanted with iPS and ES cells following MI demonstrated no evidence of teratoma formation as examined in the H&E stained sections (Figure 18).

### 8. Example 8: Transplanted iPS Cells Improve Cardiac Function

131. Echocardiography performed at 2 weeks after the LAD ligation showed significant improvement in cardiac fractional shortening in iPS transplanted hearts (MI+iPS cells; 45.4±0.7) compared with MI+ES cells, MI+H9c2 cells and MI (MI+ES cells; 41±0.9, MI+H9c2 cells; 38±0.7 and MI; 31±0.9) groups. Moreover, ES and H9c2 cell transplanted groups were significantly (p<0.05) different compared with MI hearts. Next, left ventricular interior diameter systolically (LVIDs) was significantly (p<0.05) decreased in MI+iPS and ES cell groups compared with MI and MI+H9c2 cell groups.

### 9. Example 9: Discussion

132. An objective of the present study was to generate rat cardiac iPS cells and to establish their potential to repair and regenerate infarcted myocardium. This study is the first to successfully create rat cardiac iPS cells from H9c2 cells (originally derived from embryonic rat heart tissue; Hescheler, et al., Morphological, biochemical, and electrophysiological characterization of a clonal cell (H9c2) line from rat heart. Circ Res 1991;69:1476-1486; Kimes & Brandt, Properties of a clonal muscle cell line from rat heart. Exp Cell Res 1976;98:367-381) using four sternness factors (Oct3/4, Sox2, Klf4, and c-Myc). These cardiac iPS cells exhibit numerous similar characteristics of ES cells, including; formation of ES cell like colonies, presence of four sternness factors, positive staining for alkaline phosphatase and Oct3/4 as well as their characteristics to form EBs. The cardiac iPS cell data corroborate with the undifferentiated characteristics of ES cells and iPS cells generated from human embryonic and adult fibroblasts cells (Kumar, et al., Embryonic stem cells: differentiation into cardiomyocytes and potential for heart repair and regeneration. Coron Artery Dis 2005 ;16:111-116; Thomson, et al., Embryonic stem cell lines derived from human blastocysts. Science 1998;282:1145-1147; Zhao & Daley, From fibroblasts to iPS cells: induced pluripotency by defined factors. J Cell Biochem 2008;105:949-955). Moreover, the successful reprogramming of cardiac origin H9c2 cells demonstrate a possibility to generate cardiac IPS cells from human adult or neonatal isolated cardiac myocytes.

133. Next, the procured data on cardiac myocyte derivation from human or mouse iPS cells in vitro and in vivo is from iPS cells transduced from fibroblasts using four sternness factors (Oct3/4, Sox2, KIf4, and c-Myc) or without c-Myc (Zhang, et al., Functional cardiomyocytes derived from human induced pluripotent stem cells. Circ Res 2009;104:e30-e41; Martinez-Fernandez, et al., iPS programmed without c-MYC yield proficient cardiogenesis for functional heart chimerism. Circ Res 2009;105:648-656; Narazaki, et al., Directed and systematic differentiation of cardiovascular cells from mouse induced pluripotent stem cells. Circulation 2008; 118:498-506).

134. An objective of the study was to determine whether rat cardiac iPS cells can differentiate into cardiac myocytes in the cell culture as well as repair and regenerate the infarcted mouse heart following transplantation. In this regard, EBs were developed and conclusively established that cardiac myocytes derived from iPS cells are synchronously beating and stained positive with the cardiac myocyte specific markers sarcomeric *a*-actin, and MHC antibody, MF-20. The current iPS derived cardiac myocyte differentiation data was compared and confirmed with a well established cardiomyogenesis model of ES cells (Singla & Sun, Transforming growth factor-beta2 enhances differentiation of cardiac myocytes from embryonic stem cells. Biochem Biophys Res Commun 2005;332:135-141). Moreover, recently published, mouse and human fibroblast-iPS cell derived cardiac myocytes in the cell culture system is well in accordance with the data obtained in the present study, strongly suggesting cardiac iPS cells have potential to differentiate into cardiac myocytes (Zhang, et al,, Functional cardiomyocytes derived from human induced pluripotent stem cells. Circ Res 2009;104:e30-e41; Martinez-Fernandez, et al., iPS programmed without c-MYC yield proficient cardiogenesis for functional heart chimerism. Circ Res 2009 ;105:648-656; Narazaki, et al., Directed and systematic differentiation of cardiovascular cells from mouse induced pluripotent stem cells. Circulation 2008;118:498-506). However, rat fibroblast iPS cells still need to be generated and compared with rat cardiac iPS cells to determine if one cell has a better advantage to differentiate into cardiovascular cell types compared with other cell types. Similarly, the present study opens new avenues to generate iPS cells from tissue specific iPS cell types compared with general fibroblast iPS cells. Whether generation of tissue specific iPS cells are advantageous needs further investigation.

135. Moreover, transplanted cardiac iPS cells data should be authenticated to confirm whether the cells have the potential to regenerate infarcted myocardium. The data strongly suggests that cardiac iPS cells, when inserted into the infarcted heart, lose their pluripotency and engraft into the native myocardium where the local microenvironment direct them to differentiate into cardiac myocytes. Moreover, there are concerns in stem cell transplantation studies include whether newly differentiated cells can integrate into host myocardium. The data suggests that newly formed cardiac myocytes are positive for connexin-43 and demonstrate electrical coupling. These heart regeneration data using cardiac iPS cells are important because iPS cells can easily be generated from various patient specific adult cell sources such as human cardiac myocytes. Moreover, the transplanted cardiac iPS cell regeneration data is agreeable with the performed studies in the infarcted heart using ES or fibroblast-iPS cells (Nelson, et al., Repair of acute myocardial infarction by human sternness factors induced pluripotent stem cells. Circulation 2009;120:408-416; Singla, et al., Transplantation of embryonic stem cells into the infarcted mouse heart: formation of multiple cell types. J Mol Cell Cardiol 2006;40:195-200; Behfar, et al., Stem cell differentiation requires a paracrine pathway in the heart. FASEB J 2002;16:1558-1566). Moreover, transplanted H9c2 cells were unable to demonstrate new cardiac myocyte differentiation. These data suggest that transduced cardiac iPS cells have lost parental characteristics of H9c2 cells and contain better potential to regenerate infarcted heart compared with non-transduced H9c2 cells.

136. Transplanted ES or iPS cells can form teratomas following transplantation into immune-deficient mice, which are a well established characteristic of ES cells (Thomson, et al., Embryonic stem cell lines derived from human blastocysts. Science 1998;282:1145-1147; Reubinoff, et al., Embryonic stem cell lines from human blastocysts: somatic differentiation in vitro. Nat Biotechnol 2000;18:399-404). Accordingly, teratoma formation is a major limitation following transplantation of ES cells or iPS cells in any organ. This has recently been reported that transplanted iPS cells in the infarcted myocardium of immunodeficient mice shows tumor formation compared with immunocompetent mice where no teratoma formation was observed (Nelson, et al., Repair of acute myocardial infarction by human stemness factors induced pluripotent stem cells. Circulation 2009;120:408-416). In the present study, it is firmly suggested that transplanted iPS cells in the infarcted mouse heart can successfully engraft and differentiate into cardiac myocytes with no evidence of teratoma formation. The exact reason for the absence of teratoma formation is completely unknown. However, it is possible that a low number of cardiac iPS cells were transplanted, giving the cells more exposure to cardiac release factors following MI, which may help direct differentiation into cardiac cell types. In contrast, large numbers of transplanted ES or iPS cells will form cellular clumps, which may expose them to an internal stem cell microenvironment which drives stem cell proliferation rather than cardiac cell type differentiation, leading to the formation of teratomas. Other indicators to determine whether transduced oncogene c-Myc may have lost its ability to form teratoma in these cardiac iPS cells remains unknown. The data is in an agreement with previously published studies suggesting a low number of ES or iPS cells do not form teratomas (Nelson, et al., Repair of acute myocardial infarction by human sternness factors induced pluripotent stem cells. Circulation 2009;120:408-416; Nussbaum, et al., Transplantation of undifferentiated murine embryonic stem cells in the heart: teratoma formation and immune response. FASEB J 2007;21:1345-1357; Singla, Embryonic Stem Cells in Cardiac Repair and Regeneration. Antioxid Redox Signal 2009;11:1857-1863).

137. Next, significantly improved cardiac function was observed in iPS cell transplanted hearts compared with MI controls. However, observed improved function does not correlate well with the number of newly differentiated cardiac myocytes. In this regard, this study was extended to examine whether transplanted iPS cells inhibit cardiac myocyte apoptosis and fibrosis. This is well established that apoptosis is a programmed cell death which plays a significant role in the progression of MI leading to end stage heart failure (Anversa, et al., Myocyte cell death and ventricular remodeling. Curr Opin Nephrol Hypertens 1997;6:169-176). Moreover, adverse cardiac remodeling involves fibroblast proliferation, which replaces dead apoptotic cardiac myocytes resulting in the development of fibrosis, which ultimately leads to heart dysfunction (Anversa, et al., Myocyte cell death and ventricular remodeling. Curr Opin Nephrol Hypertens 1997;6:169-176). In the present study, it is suggested that transplanted iPS cells significantly inhibit endogenous cardiac apoptosis and fibrosis in the infarcted heart. The inhibition of apoptosis and fibrosis in the present study seems to parallel the effects observed in stem cell studies previously (Singla, et al., Transplanted embryonic stem cells following mouse myocardial infarction inhibit apoptosis and cardiac remodeling. Am J Physiol Heart Circ Physiol 2007;293:H1308-H1314; Wang, et al., Combining pharmacological mobilization with intramyocardial delivery of bone marrow cells over-expressing VEGF is more effective for cardiac repair. J Mol Cell Cardiol 2006;40:736-745; Wang, et al, Chronic Preconditioning: A Novel Approach For Cardiac Protection. Am J Physiol Heart Circ Physiol 2007;292:H2300-H2305). This data suggests that a significant decrease in apoptosis and fibrosis in the MI is a common phenomenon by which cell transplantation in the infarcted heart utilizes its beneficial effects mediated through autocrine or paracrine pathways. Therefore, it is postulated that transplanted iPS cells also release autocrine or paracrine factors, which play a major role in the inhibition of apoptosis and fibrosis, which remains to be determined. Moreover, factors released from ES cells or MSC inhibit apoptosis (Singla, et al., Factors Released from Embryonic Stem Cells inhibit Apoptosis in H9c2 cells through Pl-3kinase/Akt but not ERK pathway. Am J Physiol Heart Circ Physiol;295:H907-H913; Gnecchi, et al., Paracrine action accounts for marked protection of ischemic heart by Akt-modified mesenchymal stem cells. Nat Med 2005;11:367-378; Gnecchi, et al., Evidence supporting paracrine hypothesis for Akt-modified mesenchymal stem cell-mediated cardiac protection and functional improvement. FASEB J 2006;20:661-669). However, the released factors are a distinct type of growth factor as reported (Singla, et al., Factors Released from Embryonic Stem Cells inhibit Apoptosis in H9c2 cells through Pl-3kinase/Akt but not ERK pathway. Am J Physiol Heart Circ Physiol;295:H907-H913; Gnecchi, et al, Paracrine action accounts for marked protection of ischemic heart by Akt-modified mesenchymal stem cells. Nat Med 2005;11:367-378; Gnecchi, et al, Evidence supporting paracrine hypothesis for Akt-modified mesenchymal stem cell-mediated cardiac protection and functional improvement. FASEB J 2006;20:661-669; Singla & McDonald, Factors released from embryonic stem cells inhibit apoptosis of H9c2 cells. Am J Physiol Heart Circ Physiol 2007;293:H1590-H1595). Similarly, to determine whether factors released by iPS cells are the same as ES cells or MSC needs further investigation. Overall, it is suggested that improved cardiac function in the present study is due to the formation of new cardiac myocytes as well as inhibition of adverse cardiac remodeling.

138. In conclusion, rat cardiac iPS cells are reported for the first time to be pluripotent, and able to differentiate into cardiac myocytes in the cell culture system, and regenerate infarcted myocardium. The findings also provide for the first time that two weeks post-MI iPS cells transplanted in the infarcted heart inhibit apoptosis and fibrosis. Multifactorial effects of transplanted iPS cells lead to new cardiac myocyte differentiation and a reduction in cardiac remodeling contributing to improved cardiac function.

## Claims

1. Cardiac induced pluripotent stem cells for use in the treatment of damaged cardiac tissue due to cardiac dysfunction by repairing or regenerating said damaged cardiac tissue in a subject in need thereof,
wherein the cardiac induced pluripotent stem cells are derived from cardiofibroblasts, cardiomyoblasts, or H9c2 cardiomyoblasts that stably express inserted stem-cell like factors,
wherein the inserted stem-cell like factors comprise Oct-3/4, KIf4, Sox2, and c-Myc, and
wherein cDNA sequences for the stem-cell like factors are inserted using a plasmid vector.

2. The cardiac induced pluripotent stem cells for use according to claim 1, wherein the cardiac tissue is damaged by a cardiac ischemia/reperfusion event or a congenital heart defect.

3. The cardiac induced pluripotent stem cells for use according to claim 2, wherein the ischemia/reperfusion event is myocardial infarction, myocardial ischemia, myocardial reperfusion, subendocardial ischemia, Takayasu's arteritis, atrial fibrillation, hemorrhagic strokes, an ischemia/reperfusion event occurring during cardiac surgery where a heart lung machine is used, an ischemia/reperfusion event occurring during coronary artery bypass, or ischemia/reperfusion event occurring during the preservation of an organ for transplant, or wherein the congenital heart defect is hypoplasia or pentalogy of Cantrell.

4. The cardiac induced pluripotent stem cells for use according to claim 1, wherein the cardiac induced pluripotent stem cells are administered to the subject in two equal intra-myocardial injections.

5. The cardiac induced pluripotent stem cells for use according to claim 3, wherein the ischemia/reperfusion event occurs following myocardial infarction and wherein the cardiac induced pluripotent stem cells are administered to the subject in a peri-infarct region.

6. The cardiac induced pluripotent stem cells for use according to claim 1, wherein the cardiofibroblasts, cardiomyoblasts, or H9c2 cardiomyoblasts are obtained from an autologous, allogeneic, or syngeneic source.

7. Cardiac induced pluripotent stem cells derived from cardiofibroblasts, cardiomyoblasts, or H9c2 cardiomyoblasts that stably express inserted stem-cell like factors,
wherein the inserted stem-cell like factors comprise Oct-3/4, KIf4, Sox2, and c-Myc, and
wherein cDNAs for the stem-cell like factors are inserted via a plasmid vector.

8. The cardiac induced pluripotent stem cells of claim 7, wherein the cardiofibroblasts, cardiomyoblasts, or H9c2 cardiomyoblasts are obtained from an autologous, allogeneic, or syngeneic mammalian source.

9. A method of making the cardiac induced pluripotent stem cells of claim 7, comprising:
obtaining cardiofibroblasts, cardiomyoblasts, or H9c2 cardiomyoblasts,
inserting into the cardiofibroblasts, cardiomyoblasts, or H9c2 cardiomyoblasts a plasmid vector comprising cDNA sequences for stem-cell like factors, wherein the stem-cell like factors comprise Oct-3/4, KIf4, Sox2, and c-Myc, and
stably expressing the inserted stem-cell like factors, thereby converting the cardiofibroblasts, cardiomyoblasts, or H9c2 cardiomyoblasts into cardiac induced pluripotent stem cells.

10. The method of claim 9, comprising generating the plasmid vector comprising the cDNA sequences for Oct3/4, Klf4, Sox2, and c-Myc.

11. The method of claim 10, wherein generating the plasmid vector comprising the cDNA sequences for Oct-3/4, KIf4, Sox2, and c-Myc comprises:
inserting Oct-3/4 cDNA into plasmid vector pBS-2A to yield pBS-Oct-3/4-2A;
inserting KIf4 cDNA into plasmid vector pBS-2A to yield pBS-KIf4-2A;
isolating the KIf4-2A sequence and inserting the isolated KIf4-2A sequence into the pBS-Oct-3/4-2A to yield pBS-Oct-3/4-2A-KIf4-2A;
fusing pBS-Oct-3/4-2A-KIf4-2A with Sox2 cDNA to yield pBS-Oct-3/4-2A-KIf4-2A-Sox2;
isolating the Oct-3/4-2A-KIf4-2A-Sox2 fragment and cloning the isolated fragment into plasmid vector pCX-EGFP; and
replacing EGFR in the pCX-EGFP plasmid vector with c-Myc.

## Patentansprüche

1. Kardial induzierte pluripotente Stammzellen zur Verwendung in der Behandlung von beschädigtem kardialem Gewebe infolge einer kardialen Dysfunktion durch Reparieren oder Regenerieren des beschädigten Herzgewebes in einem Patienten, der dies benötigt,
wobei die kardial induzierten pluripotenten Stammzellen entwickelt sind aus Kardiofibroblasten, Kardiomyoblasten, oder H9c2 Kardiomyoblasten, die eingefügte Stammzellen ähnliche Faktoren stabil exprimieren,
wobei die eingefügten Stammzellen ähnlichen Faktoren Oct-3/4, KIf4, Sox2, und c-Myc umfassen, und
wobei cDNA-Sequenzen für die Stammzellen ähnlichen Faktoren unter Verwendung eines Plasmidvektors eingefügt werden.

2. Kardial induzierte pluripotente Stammzellen zur Verwendung nach Anspruch 1, wobei das Herzgewebe durch eine kardiale Ischämie/ein Reperfusions-Ereignis oder einen angeborenen Herzfehler geschädigt ist.

3. Kardial induzierte pluripotente Stammzellen zur Verwendung nach Anspruch 2, wobei die Ischämie/das Reperfusions-Ereignis ein Herzinfarkt, eine myokardiale Ischämie, eine myokardiale Reperfusion, eine subendokardiale Ischämie, eine Takayasu-Arteritis, ein Vorhofflimmern, ein hämorrhagischer Schlaganfall, eine Ischämie/ein Reperfusions-Ereignis, das während einer Herzoperation auftritt, bei der eine Herz/Lungenmaschine verwendet wird, eine Ischämie/ein Reperfusions-Ereignis, das während eines koronaren arteriellen Bypass auftritt, oder eine Ischämie/ein Reperfusions-Ereignis, das während der Konservierung eines Organs zur Transplantation auftritt, ist, oder wobei der angeborene Herzfehler eine Hypoplasie oder eine Cantrell-Pentalogie ist.

4. Kardial induzierte pluripotente Stammzellen zur Verwendung nach Anspruch 1, wobei die kardial induzierten pluripotenten Stammzellen dem Patienten in zwei gleichen intramyokardialen Injektionen verabreicht werden.

5. Kardial induzierte pluripotente Stammzellen zur Verwendung nach Anspruch 3, wobei die Ischämie/das Reperfusions-Ereignis in Folge eines Herzinfarktes auftritt, und wobei die kardial induzierten pluripotenten Stammzellen dem Patienten in einer Randzone des Infarktes verabreicht werden.

6. Kardial induzierte pluripotente Stammzellen zur Verwendung nach Anspruch 1, wobei die Kardiofibroblasten, Kardiomyoblasten, oder H9c2 Kardiomyoblasten aus einer autologen, allogenen oder syngenen Quelle gewonnen sind.

7. Kardial induzierte pluripotente Stammzellen, welche abgeleitet sind aus Kardiofibroblasten, Kardiomyoblasten, oder H9c2 Kardiomyoblasten, die eingefügte Stammzellen ähnliche Faktoren stabil exprimieren,
wobei die eingefügten Stammzellen ähnlichen Faktoren Oct-3/4, KIf4, Sox2, und c-Myc umfassen, und
wobei cDNA-Sequenzen für die Stammzellen ähnlichen Faktoren unter Verwendung eines Plasmidvektors eingefügt werden.

8. Kardial induzierte pluripotente Stammzellen nach Anspruch 7, wobei die Kardiofibroblasten, Kardiomyoblasten, oder H9c2 Kardiomyoblasten aus einer autologen, allogenen oder syngenen Säugetier-Quelle gewonnen sind.

9. Verfahren zum Herstellen der kardial induzierten pluripotenten Stammzellen nach Anspruch 7, umfassend:
Gewinnen von Kardiofibroblasten, Kardiomyoblasten, oder H9c2
Einfügen eines Plasmidvektors, der cDNA-Sequenzen für Stammzellen ähnliche Faktoren umfasst, in die Kardiofibroblasten, Kardiomyoblasten, oder H9c2 Kardiomyoblasten wobei die Stammzellen ähnlichen Faktoren Oct-3/4, KIf4, Sox2, und c-Myc umfassen, und
stabiles Exprimieren der eingefügten Stammzellen ähnlichen Faktoren, wodurch die Kardiofibroblasten, Kardiomyoblasten, oder H9c2 Kardiomyoblasten in kardial induzierte pluripotente Stammzellen umgewandelt werden.

10. Verfahren nach Anspruch 9, umfassend das Erzeugen des Plasmidvektors umfassend die cDNA-Sequenzen für Oct-3/4, KIf4, Sox2, und c-Myc.

11. Verfahren nach Anspruch 10, wobei das Erzeugen des Plasmidvektors umfassend die cDNA-Sequenzen für Oct-3/4, KIf4, Sox2, und c-Myc umfasst:
Einfügen von Oct-3/4 cDNA in den Plasmidvektor pBS-2A, um pBS-Oct-3/4-2A zu gewinnen;
Einfügen von KIf4 cDNA in den Plasmidvektor pBS-2A, um pBS-KIf4-2A zu gewinnen;
Isolieren der KIf4-2A-Sequenz und Einfügen der isolierten KIf4-2A-Sequenz in den pBS-Oct-3/4-2A, um pBS-Oct-3/4-2A-KIf4-2A zu gewinnen;
Verschmelzen des pBS-Oct-3/4-2A-KIf4-2A mit Sox cDNA, um pBS-Oct-3/4-2A-KIf4-2A-Sox2 zu gewinnen;
Isolieren des Oct-3/4-2A-KIf4-2A-Sox2-Fragments und Klonen des isolierten Fragments in einen Plasmidvektor pCX-EGFP; und
Ersetzen des EGFR in dem pCX-EGFP-Plasmidvektor durch c-Myc.

## Revendications

1. Cellules souches cardiaques pluripotentes induites pour une utilisation dans le traitement de tissu cardiaque endommagé dû à un disfonctionnement cardiaque en réparant ou régénérant ledit tissu cardiaque endommagé d'un sujet le nécessitant,
dans lesquelles les cellules souches cardiaques pluripotentes induites sont dérivées de cardiofibroblastes, de cardiomyoblastes, ou de cardiomyoblastes H9c2 qui expriment de manière stable des facteurs de transcription de cellules souches insérés,
dans lesquelles les facteurs de transcription de cellules souches insérés comprennent Oct-3/4, KIf4, Sox2, et c-Myc, et
dans lesquelles des séquences d'ADNc pour les facteurs de transcription de cellules souches sont insérées en utilisant un vecteur plasmidique.

2. Cellules souches pluripotentes cardiaques induites pour une utilisation selon la revendication 1, dans lesquelles le tissu cardiaque est endommagé par un évènement ischémie/reperfusion cardiaque ou un défaut cardiaque congénital.

3. Cellules souches pluripotentes cardiaques induites pour une utilisation selon la revendication 2, dans lesquelles l'événement ischémie/reperfusion est un infarctus du myocarde, une ischémie du myocarde, une reperfusion du myocarde, une ischémie sous-endocardiaque, une artérite de Takayasu, une fibrillation auriculaire, un accident cérébrovasculaire hémorragique, un évènement ischémie/reperfusion survenant au cours d'une intervention chirurgicale cardiaque lorsqu'une machine cardiopulmonaire est utilisée, un évènement ischémie/reperfusion survenant lors d'un pontage coronarien, ou un évènement ischémie/reperfusion survenant durant la préservation d'un organe pour une transplantation, ou dans lesquelles le défaut cardiaque congénital est l'hypoplasie ou une pantalogie de Cantrell.

4. Cellules souches pluripotentes cardiaques induites pour une utilisation selon la revendication 1, dans lesquelles les cellules souches pluripotentes cardiaques induites sont administrées au sujet en deux injections égales intra-myocardiques.

5. Cellules souches cardiaques pluripotentes induites pour une utilisation selon la revendication 3, dans lesquelles l'évènement ischémie/reperfusion se produit à la suite d'un infarctus du myocarde et dans lesquelles les cellules souches pluripotentes induites sont administrées au sujet dans une région de péri-infarctus.

6. Cellules souches cardiaques pluripotentes induites pour une utilisation selon la revendication 1, dans lesquelles les cardiofibroblastes, les cardiomyoblastes, ou les cardiomyoblastes H9c2 sont obtenus à partir d'une source autologue, allogénique ou syngénique.

7. Cellules souches cardiaques pluripotentes induites dérivées de cardiofibroblastes, de cardiomyoblastes, ou de cardiomyoblastes H9c2 qui expriment de manière stable des facteurs de transcription de cellules souches insérés,
dans lesquelles les facteurs de transcription de cellules souches insérés comprennent Oct-3/4, KIf4, Sox2, et c-Myc, et
dans lesquelles des ADNc pour les facteurs de transcription de cellules souches sont insérés via un vecteur plasmidique.

8. Cellules souches cardiaques pluripotentes induites selon la revendication 7, dans lesquelles les cardiofibroblastes, les cardiomyoblastes, ou les cardiomyoblastes H9c2 sont obtenus à partir d'une source mammifère autologue, allogénique ou syngénique.

9. Procédé pour fabriquer les cellules souches cardiaques pluripotentes de la revendication 7, comprenant :
l'obtention de cardiofibroblastes, de cardiomyoblastes, ou de cardiomyoblastes H9c2,
l'insertion dans les cardiofibroblastes, les cardiomyoblastes, ou les cardiomyoblastes H9c2 d'un vecteur plasmidique comprenant des séquences d'ADNc de facteurs de transcription de cellules souches, dans lesquels les facteurs de transcription de cellules souches comprennent Oct-3/4, KIf4, Sox2, et c-Myc, et
l'expression de manière stable des facteurs de transcription de cellules souches insérés, pour ainsi convertir les cardiofibroblastes, les cardiomyoblastes, ou les cardiomyoblastes H9c2 en cellules souches cardiaques pluripotentes induites.

10. Procédé selon la revendication 9, comprenant la production d'un vecteur plasmidique comprenant les séquences d'ADNc pour Oct3/4, Klf4, Sox2, et c-Myc.

11. Procédé selon la revendication 10, dans lequel la production du vecteur plasmidique comprenant les séquences d'ADNc pour Oct-3/4, Klf4, Sox2, et c-Myc comprend :
l'insertion d'ADNc d'Oct-3/4 dans le vecteur plasmidique pBS-2A pour obtenir pBS-Oct-3/4-2A ;
l'insertion d'ADNc de Klf4 dans le vecteur plasmidique pBS-2A pour obtenir pBS-KIf4-2A ;
l'isolement de la séquence Klf4-2A et l'insertion de la séquence Klf4-2A isolée dans pBS-Oct-3/4-2A pour obtenir pBS-Oct-3/4-2A-Klf4-2A ;
la fusion de pBS-Oct-3/4-2A-Klf4-2A avec l'ADNc de Sox2 pour obtenir pBS-Oct-3/4-2A-Klf4-2A-Sox2 ;
l'isolement du fragment Oct-3/4-2A-Klf4-2A-Sox2 et le clonage du fragment isolé dans le vecteur plasmidique pCX-EGFP ; et
le remplacement d'EGFR dans le vecteur plasmidique pCX-EGFP par c-Myc.
